(19) Europäisches Patentamt
European Patent Office
Office européen des brevets

(11) Publication number : **0 389 423 B1**

# (12) EUROPEAN PATENT SPECIFICATION

(45) Date of publication of patent specification : **27.04.94 Bulletin 94/17**

(51) Int. Cl.$^5$ : **C07D 211/94,** C08K 5/3435

(21) Application number : **90810191.8**

(22) Date of filing : **13.03.90**

## (54) Peroxide compounds containing hindered amine moieties with low basicity.

(30) Priority : **21.03.89 US 326353**

(43) Date of publication of application : **26.09.90 Bulletin 90/39**

(45) Publication of the grant of the patent : **27.04.94 Bulletin 94/17**

(84) Designated Contracting States : **DE FR GB IT**

(56) References cited :
**EP-A- 0 056 699**
**EP-A- 0 082 738**
**EP-A- 0 233 476**

(73) Proprietor : **CIBA-GEIGY AG**
**Klybeckstrasse 141**
**CH-4002 Basel (CH)**

(72) Inventor : **Seltzer, Raymond**
**11 Angus Lane**
**New City, N.Y. 10956 (US)**
Inventor : **Winter, Roland A.E.**
**23 Banksville Road**
**Armonk, N.Y. 10504 (US)**
Inventor : **Schirmann, Peter J.**
**89 Eunice Avenue**
**Fairfeld, C.T. 06430 (US)**

EP 0 389 423 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The instant invention discloses hindered amine light stabilizers which combine low basicity with a peroxy group in the same molecule.

The initiation of the polymerization of acrylic monomers with peroxy esters bearing hindered amine light stabilizing substituents is described by P.A. Callais et al in a paper presented in February 1988 at the "Waterborne and Higher Solids Coating Symposium" in New Orleans and published in Modern Paint and Coatings, 78 (9), 41 (1988).

Peroxides as free radical initiators containing hindered amine moieties is described in EP-A-233,476.

The combination of ultraviolet stabilizers (UV absorbers) with free radical initiating moieties (azo derivatives and peroxide compounds) are disclosed in United States Patent Nos. 3,956,269; 4,042,773; 4,045,426; 4,045,427; 4,055,714 and 4,129,586.

The instant invention overcomes the drawbacks of the prior art materials which combine hindered amines with high basicity with peroxy groups.

The high basicity can neutralize acid catalysts that are commonly used in thermosetting resins thus causing cure inhibition. In other applications, the high basicity of many hindered amines can lead to undesired complexing and deactivation of metal ions which are used as catalysts for oxidative curing processes as well as undesired interactions with some pigment systems.

U.S. Patent No. 4,822,883 describes peroxide free radical initiators containing hindered amine light stabilizer groups, but said hindered amines are not of low basicity.

The thermal cleavage of the peroxy moiety in the molecule results in the formation of free radicals which can be used to initiate free radical polymerization of ethylenically unsaturated monomers.

Another application involves the grafting of the stabilizer to existing substrates including a variety of polymers.

In either of these two situations, the instant light stabilizing hindered amine moiety becomes substantially chemically bonded to the substrate and becomes concomitantly resistant to migration, exudation, leaching, sublimation, volatilization or any process which is prone to remove an additive physically from the substrate it is supposed to protect.

More particularly, the instant invention pertains to a compound which is a free radical initiator which also contains a hindered amine light stabilizing moiety having low basicity, which compound has the formula I

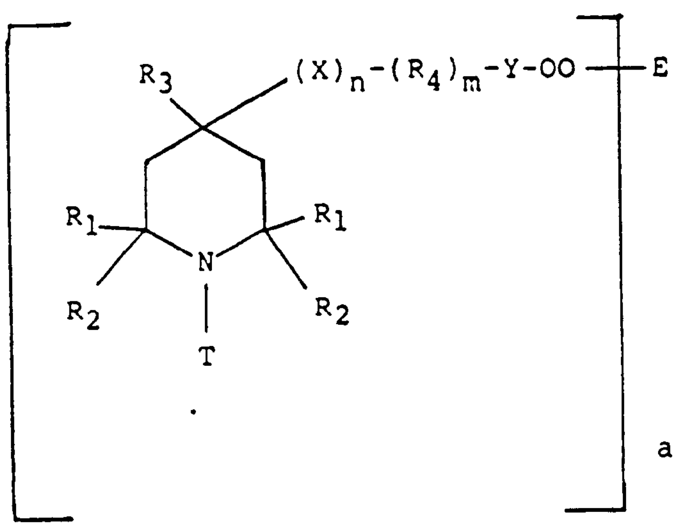

(I)

a is 1 or 2,

n and m are independently 0 or 1,

$R_1$ and $R_2$ are independently alkyl of 1 to 4 carbon atoms, or $R_1$ and $R_2$ together are pentamethylene,

$R_3$ is hydrogen, alkyl of 1 to 8 carbon atoms, alkenyl of 2 to 8 carbon atoms, alkynyl of 2 to 8 carbon atoms, cycloalkyl of 5 to 6 carbon atoms, alkoxy of 1 to 8 carbon atoms, alkoxycarbonyl of 2 to 7 carbon atoms, aryl of 6 to 10 carbon atoms, aralkyl of 7 to 15 carbon atoms, alkanoyl of 1 to 8 carbon atoms, aroyl of 7 to 16 carbon atoms, alkanoyloxy of 1 to 7 carbon atoms, or aroyloxy of 6 to 10 carbon atoms, or $R_3$ together with $R_4$ form a cyclic structure of 5 to 7 atoms,

X is -O-, -S-, -NG-, -CO-, -SO-, $-SO_2-$, -OCO-, -OSO-, $-OSO_2-$, -NG-CO-, -NHCONH- or -OCO-O- where

G is hydrogen, alkyl of 1 to 8 carbon atoms, alkenyl of 2 to 8 carbon atoms, alkynyl of 2 to 8 carbon atoms, cycloalkyl of 5 to 6 carbon atoms, aryl of 6 to 10 carbon atoms, alkanoyl of 1 to 8 carbon atoms or G and $R_4$ together form a cyclic structure of 5 to 7 carbon atoms,

$R_4$ is a diradical which is alkylene of 1 to 20 carbon atoms, arylene of 6 to 10 carbon atoms, cycloalkylene of 3 to 10 carbon atoms, aralkylene of 7 to 20 carbon atoms, alkynylene of 2 to 10 carbon atoms, alkadiynylene of 4 to 10 carbon atoms, alkenylene of 3 to 11 carbon atoms, alkadienylene of 5 to 11 carbon atoms, or said diradical interrupted by one or more oxygen, sulfur or nitrogen atoms,

Y is -CO-, $-SO_2-$, $-CR_5R_6-$, -O-R-, -NG-R- or -OCO-, where $R_5$ and $R_6$ are independently alkyl of 1 to 10 carbon atoms, aryl of 6 to 10 carbon atoms, alkynyl of 2 to 10 carbon atoms, alkenyl of 2 to 8 carbon atoms or cycloalkyl of 5 to 6 carbon atoms, or $R_5$ and $R_6$ together are alkylene of 4 to 9 carbon atoms, and when E is tert-alkyl, tert-cycloalkyl or tert-aralkyl, $R_6$ is also -O-O-E, and A is di-tert-alkylene of 7 to 15 carbon atoms, di-tert-aralkylene of 12 to 20 carbon atoms, alkanedioyl of 3 to 12 carbon atoms or arene dicarbonyl of 8 to 16 carbon atoms,

E, when a is 1, is hydrogen, alkanoyl of 2 to 20 carbon atoms, aroyl of 7 to 20 carbon atoms, tert-alkyl of 4 to 12 carbon atoms, tert-cycloalkyl of 4 to 12 carbon atoms, tert-aralkyl of 9 to 15 carbon atoms, alkoxycarbonyl of 2 to 20 carbon atoms, carbamoyl, phenylcarbamoyl, alkylcarbamoyl of 2 to 13 carbon atoms, cycloalkylcarbamoyl of 4 to 13 carbon atoms, alpha-hydroxyalkyl of 2 to 10 carbon atoms, alpha-hydroxycycloalkyl of 3 to 10 carbon atoms, alkylsulfonyl of 4 to 20 carbon atoms, cycloalkylsulfonyl of 3 to 12 carbon atoms, tert-alkoxyalkyl of 4 to 20 carbon atoms, tert-alkoxycycloalkyl of 4 to 20 carbon atoms, monovalent organometal, or the radical of formula II

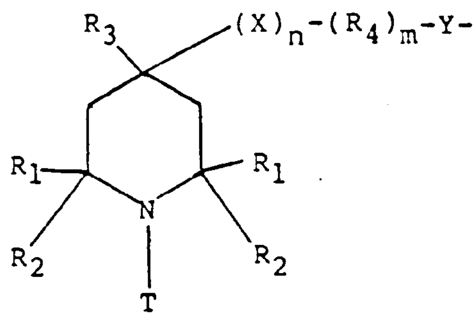

(II)

, or

E, when a is 2, is di-tert-alkylene of 7 to 15 carbon atoms, di-tert-alkenylene of 8 to 16 carbon atoms, di-tert-alkynylene of 8 to 16 carbon atoms, di-tert-aralkylene of 12 to 20 carbon atoms, alkanedioyl of 3 to 12 carbon atoms,arenedicarbonyl of 8 to 16 carbon atoms araralkanedicarbonyl of 9 to 18 carbon atoms, and

T is formyl, $-O-T_1$, or $-OCO-T_2$, where

$T_1$ is alkyl of 1 to 36 carbon atoms, alkenyl of 2 to 18 carbon atoms, alkynyl of 2 to 18 carbon atoms, aralkyl of 7 to 15 carbon atoms, cycloalkyl of 5 to 12 carbon atoms, cycloalkenyl of 5 to 12 carbon atoms, a radical of a saturated or unsaturated bicyclic or tricyclic hydrocarbon of 7 to 12 carbon atoms, or aryl of 6 to 10 carbon atoms or said aryl substituted by alkyl, and

$T_2$ is alkyl of 1 to 18 carbon atoms, alkoxy of 1 to 18 carbon atoms, phenyl or said phenyl substituted by hydroxy, alkyl or alkoxy; or amino or said amino mono- or disubstituted by alkyl or phenyl.

Preferably $R_1$ and $R_2$ are each methyl.

$R_3$ is preferably hydrogen or alkoxycarbonyl of 2 to 5 carbon atoms.

X is preferably -O-, -S-, -NG-, -OCO-O-, -NGCOO-, -OCO- or -CO-.

$R_4$ is preferably alkylene of 1 to 8 carbon atoms, arylene of 6 to 10 carbon atoms, aralkylene of 8 to 16 carbon atoms or cycloalkylene of 4 to 8 carbon atoms.

When a is 1, E is preferably alkanoyl of 2 to 10 carbon atoms, aroyl of 7 to 10 carbon atoms, tert-alkyl of 4 to 8 carbon atoms, tert-aralkyl of 9 to 16 carbon atoms or the radical of formula II.

When a is 2, E is preferably di-tert-alkylene of 8 to 12 carbon atoms, di-tert-aralkylene of 12 to 15 carbon atoms or alkanedioyl of 3 to 6 carbon atoms.

G is preferably hydrogen, alkyl of 1 to 4 carbon atoms, cyclohexyl or phenyl.

$R_5$ is preferably alkyl of 1 to 6 carbon atoms, aryl of 6 to 10 carbon atoms or cycloalkyl of 5 to 6 carbon atoms.

T is preferably formyl, $-OT_1$ or $-OCOT_2$ where

$T_1$ is alkyl of 1 to 18 carbon atoms, alkenyl of 2 to 3 carbon atoms, propargyl, alpha-methylbenzyl or

cyclohexyl, and $T_2$ is alkyl of 1 to 18 carbon atoms.

Most preferably $T_1$ is methyl, heptyl, octyl, nonyl or cyclohexyl.

Most preferably $T_2$ is alkyl of 1 to 12 carbon atoms.

Most preferably $R_3$ is hydrogen.

X is most preferably -O-, -NG-, -OCO-O, -NG-COO- or -OCO-.

$R_4$ is most preferably alkylene of 1 to 6 carbon atoms, phenylene, aralkylene of 9 to 12 carbon atoms or cycloalkylene of 5 to 7 carbon atoms.

Y is most preferably -CO-, $-CR_5R_6-$ or -OCO-.

When a is 1, E is most preferably alkanoyl of 2 to 10 carbon atoms, benzoyl, tert-alkyl of 4 to 6 carbon atoms, tert-aralkyl of 9 to 12 carbon atoms or a radical of formula II.

When a is 2, E is most preferably di-tert-alkylene of 8 to 10 carbon atoms, di-tert-aralkylene of 12 carbon atoms or alkanedioyl of 4 to 6 carbon atoms.

G is most preferably hydrogen or alkyl of 1 to 4 carbon atoms.

$R_5$ is most preferably alkyl of 1 to 4 carbon atoms, phenyl or cyclohexyl.

The instant invention also pertains to a process of preparing a homo-or copolymer containing a hindered amine light stabilizer moiety chemically bonded to the backbone of said polymer which process comprises polymerizing one or more ethylenically unsaturated monomer capable of being polymerized by free radicals in the presence of an effective initiating amount of a compound of formula I.

The instant compounds can be prepared by methods well known in the art as outlined in EP-A-233,476.

The intermediates used to make the instant compounds are generally items of commerce.

Unsaturated polyester resins that can be cured by the compounds of this invention usually include an unsaturated polyester and one or more polymerizable monomers. The unsaturated polyesters are, for instance, obtained by esterifying at least one ethylenically unsaturated di-or polycarboxylic acid, anhydride, or acid halide, such as maleic acid, fumaric acid, glutaconic acid, itaconic acid, mesaconic acid, citraconic acid, allylmalonic acid, allylsuccinic acid, tetrahydrophthalic acid and others with saturated or unsaturated di-or polyols, such as ethylene glycol, diethylene glycol, triethylene glycol, 1,2-and 1,3-propanediols, 1,2-, 1,3-and, 1,4-butanediols, 2,2-dimethyl-1,3-propanediols, 2,2-dimethyl-1,3-propanediol, 2-hydroxymethyl-2-methyl-1,3-propanediol, 2-buten-1,4-diol, 2,2,4-trimethyl-1,3-pentanediol, glycerol, pentaerythritol, mannitol and others. Mixtures of such polyacids and/or mixtures of such polyalcohols may also be used. The unsaturated di-or polycarboxylic acids may be partially replaced by saturated polycarboxylic acids, such as adipic acid, succinic acid, sebacic acid, and others and/or by aromatic polycarboxylic acids, such as phthalic acid, trimellitic acid, pyromellitic acid, isophthalic acid, and terephthalic acid. The acids used may be substituted by groups such as halogen. Examples of such suitable halogenated acids are, for instance, tetrachlorophthalic acid, 5,6-dicarboxy-1,2,3,4,7,7-hexachlorobicyclo[2.2.1]heptane, and others.

The other component of the unsaturated polyester resin, the polymerizable monomer or monomers, are preferably ethylenically unsaturated monomers, such as styrene, chlorostyrene, vinyltoluene, divinylbenzene, alpha-methylstyrene, diallyl maleate, diallyl phthalate, dibutyl fumarate, acrylonitrile, triallyl phosphate, triallyl cyanurate, methyl acrylate, methyl methacrylate, n-butyl methacrylate, ethyl acrylate, and others or mixtures thereof, which are copolymerizable with said polyesters.

A preferred unsaturated polyester resin contains as the polyester component the esterificaton product of 1,2-propylene glycol (a polyalcohol), maleic anhydride (an anhydride of an unsaturated polycarboxylic acid) and phthalic anhydride (an anhydride of an aromatic dicarboxylic acid) as well as the monomer component, styrene.

Other unsaturated polyester resins that are useful in the practice of this invention are unsaturated vinyl ester resins, consisting of a vinyl ester resin component and one or more polymerizable monomer components. The vinyl ester resin component can be made by reacting a chloroepoxide such as epichlorohydrin with appropriate amounts of a glycol such as bisphenol A (2,2-di-(4-hydroxyphenyl)-propane, in the presence of a base such as sodium hydroxide, to yield a condensation product having terminal epoxy groups derived from the epichlorohydrin. Subsequent reaction of the condensation product with polymerizable unsaturated carboxylic acids in the presence or absence of acidic or basic catalysts, results in the formation of a vinyl ester terminated resin component. Normally, styrene is added as the polymerizable mononer component to complete the preparation of the unsaturated vinyl ester resin.

Temperatures of about 20° to 200°C and peroxide levels of about 0.05 to 5% or more by weight of curable unsaturated polyester resin are normally employed in the curing process. The unsaturated polyester resins described above can be filled with various materials such as sulfur, glass fibers, carbon blacks, silicas, metal silicates, clays, metal carbonates, antioxidants, heat and light stabilizers, sensitizers, dyes, pigments, accelerators, metal oxides such as zinc oxide, blowing agents, etc.

The hindered amine-peroxide compounds of the present invention are useful as free radical initiators for

the polymerization or copolymerization of ethylenically unsaturated monomers or mixtures thereof at suitable temperatures and pressures. The compounds are useful not only in conventional isothermal polymerization processes but also in processes in which two or more increasing temperature steps are employed or a continuous increase in temperature is employed. Ethylenically unsaturated monomers include: olefins such as ethylene, propylene, styrene, alpha-methyl styrene, chlorostyrene, vinyl benzyl chloride, vinyl toluene, vinyl pyridine, divinyl benzene; diolefins such as 1,3-butadiene, isoprene and chloroprene; vinyl esters such as vinyl acetate, vinyl propionate, vinyl laurate, vinyl benzoate or divinyl carbonate; unsaturated nitriles such as acrylonitrile and methacrylonitrile; acrylic acid, methacrylic acid and their esters and amides, such as methyl ethyl, n-butyl and 2-ethylhexyl acrylates and methacrylates and acrylamide and methacrylamide; maleic anhydride; maleimide and N-substituted derivatives thereof such as n-phenylmaleimide; maleic and fumaric acids and their esters; vinyl halo and vinylidene halo compounds such as vinyl chloride, vinyl fluoride, vinylidene chloride and vinylidene fluoride; perhalo olefins such as tetrafluoroethylene, hexafluoropropylene and chlorotrifluoroethylene; vinyl esters such as methyl vinyl ether, ethyl vinyl ether, n-butyl vinyl ether; allyl esters such as allyl acetate, allyl benzoate, diallyl phthalate, allyl ethyl carbonate, triallyl phosphate, triallyl cyanurate, diallyl fumarate, diallyl succinate, and diallyl carbonate; acrolein; methyl vinyl ketone; and mixtures thereof.

Temperatures of 30° to 250°C, preferably 40° to 200°C, and peroxide levels of 0.005 to 3%, preferably 0.01 to 1%, by weight, based on monomer, are normally employed in the conventional polymerization or in the increasing temperature polymerization processes. Polymerization can be carried out in solution where solvents such as toluene may be used. Bulk, solution, suspension, or emulsion polymerization processes may be employed. The hindered amine-peroxide composition of this invention may be employed in these vinyl polymerization processes alone or together with other peroxides and azo initiators.

The hindred amine-peroxide compounds of this invention are also useful for producing high impact polymers such as high impact polystyrene by initiating grafting of a monomer onto the backbone of elastomers (rubbers) such as polybutadienes, styrene-butadiene-styrene triblock copolymers, ethylene-propylene-diene terpolymers, etc. This composition is also useful with lower amounts of the rubber to produce high impact resistant polymers having impact resistance comparable to high impact polymers produced with larger amounts of rubber and conventional initiator systems. The above described vinyl polymerization conditions and initiator levels and up to 15% by weight of rubber (based on monomer) may be used for producing high impact polymers.

The ethylenically unsaturated comonomers may also contain a UV-absorbing moiety such as hydroxyphenyl substituted benzotriazole or s-triazine, a hydroxy substituted benzophenone, an oxanilide or alpha-cyanocinnamate or a hindred amine light stabilizer moiety. Examples of such ethylenically unsaturated UV-absorbers are described in a number of United States patents which are hereby incorporated into this application by reference.

Ethylenically unsaturated UV absorbers are discribed in a number of U.S. Patents. Acrylated benzotriazoles are described in U.S. Patent Nos. 4,413,095; 4,716,234; 4,785,063 and 4,803,254. Acryloxyalkyl benzotriazoles are described in U.S. Patent No. 4,260,768. Vinyl substituted benzotriazoles are discribed in U.S. Patent No. 4,508,882. U.S. Patent No. 3,493,539. Acrylated benzophenones are described in U.S. Patent No. 4,310,650.

Although the instant application emphasizes the 2,2,6,6-tetraalkylpiperidine structure, it is to be noted that the invention also relates to compounds wherein the following tetraalkyl substituted piperazine or piperazinone moieties are substituted for the above-noted tetraalkylpiperidine moiety:

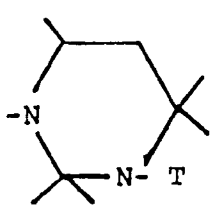

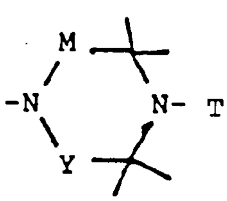

wherein M and Y are independently methylene or carbonyl, preferably M being methylene and Y being carbonyl. It is understood that the identified substituents applicable to such compounds are those which are appropriate for substitution on the ring nitrogen atoms.

The hindered amine compounds of formula I can also be used as stabilising additives for polymers which are degradable by UV light.

Substrates in which the compounds of this invention are particularly useful are polyolefins such as polyethylene and polypropylene; polystyrene, including especially impact polystyrene; ABS resin; elastomers such as e.g. butadiene rubber, EPM, EPDM, SBR and nitrile rubber.

In general polymers which can be stabilized include

1. Polymers of monoolefins and diolefins, for example polyethylene (which optionally can be crosslinked), polypropylene, polyisobutylene, polybutene-1, polymethylpentene-1, polyisoprene or polybutadiene, as well as polymers of cycloolefins, for instance of cyclopentene or norbornene.

2. Mixtures of the polymers mentioned under 1), for example mixtures of polypropylene with polyisobutylene.

3. Copolymers of monoolefins and diolefins with each other or with other vinyl monomers, such as, for example, ethylene/propylene, propylene/butene-1, propylene/isobutylene, ethylene/butene-1, propylene/butadiene, isobutylene/isoprene, ethylene/alkyl acrylates, ethylene/alkyl methacrylates, ethylene/vinyl acetate or ethylene/acrylic acid copolymers and their salts (ionomers) and terpolymers of ethylene with propylene and a diene, such as hexadiene, dicyclopentadiene or ethylidene-norbornene.

4. Polystyrene, poly-(p-methylstyrene).

5. Copolymers of styrene or methylstyrene with dienes or acrylic derivatives, such as, for example, styrene/butadiene, styrene/acrylonitrile, styrene/ethyl methacrylate, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block polymers of styrene, such as, for example, styrene/butadiene/styrene, styrene/isoprene/styrene, styrene/ethylene/butylene/styrene or styrene/ethylene/propylene/styrene.

6. Graft copolymers of styrene, such as, for example, styrene on polybutadiene, styrene and acrylonitrile on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.

7. Halogen-containing polymers, such as polychloroprene, chlorinated rubbers, chlorinated or sulfochlorinated polyethylene, epichlorohydrin homo- and copolymers, polymers from halogen-containing vinyl compounds, as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate, vinylidene chloride/vinyl acetate copolymers, or vinyl fluoride/vinyl ether copolymers.

8. Polymers which are derived from $\alpha,\beta$-unsaturated acids and derivatives thereof, such as polyacrylates and polymethacrylates, polyacrylamide and polyacrylonitrile.

9. Copolymers from the monomers mentioned under 8) with each other or with other unsaturated monomers, such as, for instance, acrylonitrile/butadiene, acrylonitrile/alkyl acrylate, acrylonitrile/alkoxyalkyl acrylate or acrylonitrile/vinyl halogenide copolymers or acrylonitrile/alkyl methacrylate/butadiene terpolymers.

10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinylbutyral, polyallyl phthalate or polyallyl-melamine.

11. Homopolymers and copolymers of cyclic ethers, such as polyalkylene glycols, polyethylene oxide, polypropylene oxide or copolymers thereof with bis-glycidyl ethers.

12. Polyacetals, such as polyoxymethylene and those polyoxymethylenes which contain ethylene oxide as comonomer.

13. Polyphenylene oxides and sulfides, and mixtures of polyphenylene oxides with polystyrene.

14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).

15. Polyamides and copolyamides which are derived from diamines and dicarboxylic acids and/or from aminocarboxylic acids or the corresponding lactams, such as polyamide 4, polyamide 6, polyamide 6/6, polyamide 6/10, polyamide 11, polyamide 12, poly-2,4,4-trimethylhexamethylene terephthalamide, poly-p-phenylene terephthalamide or poly-m-phenylene isophthalamide, as well as copolymers thereof with polyethers, such as for instance with polyethylene glycol, polypropylene glycol or polytetramethylene glycols.

16. Polyureas, polyimides and polyamide-imides.

17. Polyesters which are derived from dicarboxylic acids and diols and/or from hydroxycarboxylic acids or the corresponding lactones, such as polyethylene terephthalate, polybutylene terephthalate, poly-1,4-dimethylol-cyclohexane terephthalate, poly-[2,2-(4-hydroxyphenyl)propane] terephthalate and polyhydroxybenzoates as well as block-copolyether-esters derived from polyethers having hydroxyl end groups.

18. Polycarbonates.

19. Polysulfones, polyethersulfones and polyetherketones.

20. Crosslinked polymers which are derived from aldehydes on the one hand and phenols, ureas and melamines on the other hand, such as phenol/formaldehyde resins, urea/formaldehyde resins and melamine/formaldehyde resins.

21. Drying and non-drying alkyd resins.

22. Unsaturated polyester resins which are derived from copolyesters of saturated and unsaturated dicarboxylic acids with polyhydric alcohols and vinyl compounds as crosslinking agents, and also halogen-containing modifications thereof of low flammability.

23. Thermosetting acrylic resins, derived from substituted acrylic esters, such as epoxy-acrylates, urethane-acrylates or silicone -acrylates.

24. Alkyd resins, polyester resins or acrylate resins in admixture with melamine resins, urea resins, polyisocyanates or epoxide resins as crosslinking agents.

25. Crosslinked epoxide resins which are derived from polyepoxides, for example from bis-glycidyl ethers or from cycloaliphatic diepoxides.

26. Natural polymers, such as cellulose, rubber, gelatin and derivatives thereof which are chemically modified in a polymer homologous manner, such as cellulose acetates, cellulose propionates and cellulose butyrates, or the cellulose ethers, such as methyl cellulose.

27. Mixtures of polymers as mentioned above, for example PP/EPDM, Polyamide 6/EPDM or ABS, PVC/EVA, PVC/ABS, PVC/MBS, PC/ABS, PBTP/ABS.

28. Naturally occuring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellitates) and also mixtures of synthetic esters with mineral oils in any weight ratios, which materials may be used as plasticizers for polymers or as textile spinning oils, as well as aqueous emulsions of such materials.

29. Aqueous emulsions of natural or synthetic rubber, e.g. natural latex or latices of carboxylated styrene/butadiene copolymers.

30. Polysiloxanes such as the soft, hydrophilic polysiloxanes described, for example, in U.S. Patent No. 4,259,467; and the hard polyorganosiloxanes described, for example, in U.S. Patent No. 4,355,147.

31. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.

32. Radiation curable compositions containing ethylenically unsaturated monomers or oligomers and a polyunsaturated aliphatic oligomer.

33. Epoxymelamine resins such as light-stable epoxy resins crosslinked by an epoxy functional coetherified high solids melamine resin such as LSE 4103 (Monsanto).

In general, the compounds of formula I are employed in from about 0.01 to about 5% by weight of the stabilized composition, although this will vary with the particular substrate and application. An advantageous range is from about 0.5 to about 2%, and especially 0.1 to about 1%.

The stabilizers of the instant invention may readily be incorporated into the organic polymers by conventional techniques, at any convenient stage prior to the manufacture of shaped articles therefrom. For example, the stabilizer may be mixed with the polymer in dry powder form, or a suspension or emulsion of the stabilizer may be mixed with a solution, suspension, or emulsion of the polymer. The resulting stabilized polymer compositions of the invention may optionally also contain various conventional additives, such as the following.

1. Antioxidants

1.1. Alkylated monophenols, for example,
2,6-di-tert-butyl-4-methylphenol
2-tert-butyl-4,6-dimethylphenol
2,6-di-tert-butyl-4-ethylphenol
2,6-di-tert-butyl-4-n-butylphenol
2,6-di-tert-butyl-4-i-butylphenol
2,6-di-cyclopentyl-4-methylphenol
2-($\alpha$-methylcyclohexyl)-4,6-dimethylphenol
2,6-di-octadecyl-4-methylphenol
2,4,6-tri-cyclohexylphenol
2,6-di-tert-butyl-4-methoxymethylphenol

1.2. Alkylated hydroquinones, for example,
2,6-di-tert-butyl-4-methoxyphenol

2,5-di-tert-butyl-hydroquinone
2,5-di-tert-amyl-hydroquinone
2,6-diphenyl-4-octadecyloxyphenol

1.3. Hydroxylated thiodiphenyl ethers, for example

2,2'-thio-bis-(6-tert-butyl-4-methylphenol)
2,2'-thio-bis-(4-octylphenol)
4,4'-thio-bis-(6-tert-butyl-3-methylphenol)
4,4'-thio-bis-(6-tert-butyl-2-methylphenol)

1.4. Alkylidene-bisphenols, for example,

2,2'-methylene-bis-(6-tert-butyl-4-methylphenol)
2,2'-methylene-bis-(6-tert-butyl-4-ethylphenol)
2,2'-methylene-bis-[4-methyl-6-(α-methylcyclohexyl)-phenol]
2,2'-methylene-bis-(4-methyl-6-cyclohexylphenol)
2,2'-methylene-bis-(6-nonyl-4-methylphenol)
2,2'-methylene-bis-[6-(α-methylbenzyl)-4-nonylphenol]
2,2'-methylene-bis-[6-(α,α-dimethylbenzyl)-4-nonylphenol]
2,2'-methylene-bis-(4,6-di-tert-butylphenol)
2,2'-ethylidene-bis-(4,6-di-tert-butylphenol)
2,2'-ethylidene-bis-(6-tert-butyl-4-isobutylphenol)
4,4'-methylene-bis-(2,6-di-tert-butylphenol)
4,4'-methylene-bis-(6-tert-butyl-2-methylphenol)
1,1-bis-(5-tert-butyl-4-hydroxy-2-methylphenyl-butane
2,6-di-(3-tert-butyl-5-methyl-2-hydroxybenzyl)-4-methylphenol
1,1,3-tris-(5-tert-butyl-4-hydroxy-2-methylphenyl)-butane
1,1-bis-(5-tert-butyl-4-hydroxy-2-methylphenyl)-3-n-dodecylmercaptobutane
ethyleneglycol bis-[3,3-bis-(3'-tert-butyl-4'-hydroxyphenyl)-butyrate]
di-(3-tert-butyl-4-hydroxy-5-methylphenyl)-dicyclopentadiene
di-[2-(3'-tert-butyl-2'-hydroxy-5'-methyl-benzyl)-6-tert-butyl-4-methylphenyl] terephthalate.

1.5. Benzyl compounds, for example,

1,3,5-tri-(3,5-di-tert-butyl-4-hydroxybenzyl)-2,4,6-trimethylbenzene
di-(3,5-di-tert-butyl-4-hydroxybenzyl) sulfide
3,5-di-tert-butyl-4-hydroxybenzyl-mercapto-acetic acid isooctyl ester
bis-(4-tert-butyl-3-hydroxy-2,6-dimethylbenzyl)dithiol terephthalate
1,3,5-tris-(3,5-di-tert-butyl-4-hydroxybenzyl) isocyanurate
1,3,5-tris-(4-tert-butyl-3-hydroxy-2,6-dimethylbenzyl) isocyanurate
3,5-di-tert-butyl-4-hydroxybenzyl-phosphoric acid dioctadecyl ester
3,5-di-tert-butyl-4-hydroxybenzyl-phosphoric acid monoethyl ester, calcium-salt

1.6. Acylaminophenols, for example,

4-hydroxy-lauric acid anilide
4-hydroxy-stearic acid anilide
2,4-bis-octylmercapto-6-(3,5-tert-butyl-4-hydroxyanilino)-s-triazine
octyl-N-(3,5-di-tert-butyl-4-hydroxyphenyl)-carbamate

1.7. Esters of β-(3,5-di-tert-butyl-4-hydroxyphenyl)-propionic acid with monohydric or polyhydric alcohols, for example,

| | |
|---|---|
| methanol | diethylene glycol |
| octadecanol | triethylene glycol |
| 1,6-hexanediol | pentaerythritol |
| neopentyl glycol | tris-hydroxyethyl isocyanurate |
| thiodiethylene glycol | di-hydroxyethyl oxalic acid diamide |

1.8. Esters of β-(5-tert-butyl-4-hydroxy-3-methylphenyl)-propionic acid with monohydric or polyhydric al-

cohols, for example,

| | |
|---|---|
| methanol | diethylene glycol |
| octadecanol | triethylene glycol |
| 1,6-hexanediol | pentaerythritol |
| neopentyl glycol | tris-hydroxyethyl isocyanurate |
| thiodiethylene glycol | di-hydroxyethyl oxalic acid diamide |

1.9. Amides of β-(3,5-di-tert-butyl-4-hydroxyphenyl)-propionic acid for example,
N,N'-di-(3,5-di-tert-butyl-4-hydroxyphenylpropionyl)-hexamethylenediamine
N,N'-di-(3,5-di-tert-butyl-4-hydroxyphenylpropionyl)-trimethylenediamine
N,N'-di-(3,5-di-tert-butyl-4-hydroxyphenylpropionyl)-hydrazine

2. UV absorbers and light stabilizers

2.1. 2-(2'-Hydroxyphenyl)-benzotriazoles, for example, the 5'-methyl-, 3',5'-di-tert-butyl-, 5'-tert-butyl-, 5'-(1,1,3,3-tetramethylbutyl)-, 5-chloro-3',5'-di-tert-butyl-, 5-chloro-3'-tert-butyl-5'-methyl-, 3' -sec-butyl-5'-tert-butyl-, 4'-octoxy, 3',5'-di-tert-amyl-, 3',5'-bis-(α,α-dimethylbenzyl), 3'-tert-butyl-5'-(2-(omega-hydroxy-octa-(ethyleneoxy)carbonyl-ethyl)-, 3'-dodecyl-5'-methyl-, and 3'-tert-butyl-5'-(2-octyloxycarbonyl)ethyl-, and dodecylated-5'-methyl derivatives.

2.2. 2-Hydroxy-benzophenones, for example, the 4-hydroxy-, 4-methoxy-, 4-octoxy, 4-decyloxy-, 4-dodecyloxy-, 4-benzyloxy, 4,2',4'-trihydroxy- and 2'-hydroxy-4,4'-dimethoxy derivatives.

2.3. Esters of optionally substituted benzoic acids for example, phenyl salicylate, 4-tert-butylphenyl salicylate, octylphenyl salicylate, dibenzoylresorcinol, bis-(4-tert-butylbenzoyl)-resorcinol, benzoylresorcinol, 3,5-di-tert-butyl-4-hydroxybenzoic acid 2,4-di-tert-butylphenyl ester and 3,5-di-tert-butyl-4-hydroxybenzoic acid hexadecyl ester.

2.4. Acrylates, for example,α-cyano-β,β-diphenylacrylic acid ethyl ester or isooctyl ester, α-carbomethoxy-cinnamic acid methyl ester,α-cyano-β-methyl-p-methoxy-cinnamic acid methyl ester or butyl ester,α-carbomethoxy-p-methoxy-cinnamic acid methyl ester, N-(β-carbomethoxy-β-cyanovinyl)-2-methyl-indoline.

2.5 Nickel compounds, for example, nickel complexes of 2,2'-thio-bis-[4-(1,1,3,3-tetramethylbutyl)-phenol], such as the 1:1 or 1:2 complex, optionally with additional ligands such as n-butylamine, triethanolamine or N-cyclohexyl-diethanolamine, nickel dibutyldithiocarbamate, nickel salts of 4-hydroxy-3,5-di-tert-butylbenzylphosphonic acid monoalkyl esters, such as of the methyl, ethyl or butyl ester, nickel complexes of ketoximes such as of 2-hydroxy-4-methyl-phenyl undecyl ketoxime, nickel complexes of 1-phenyl-4-lauroyl-5-hydroxy-pyrazole, optionally with additional ligands.

2.6. Sterically hindered amines, for example bis-(2,2,6,6-tetramethylpiperidyl) sebacate, bis-(1,2,2,6,6-pentamethylpiperidyl) sebacate, n-butyl-3,5-di-tert.butyl-4-hydroxybenzyl malonic acid bis-(1,2,2,6,6-pentamethylpiperidyl)ester, condensation product of 1-hydroxyethyl-2,2,6,6-tetramethyl-4-hydroxy piperidine and succinic acid, condensation product of N,N'-(2,2,6,6-tetramethylpiperidyl)-hexamethylenediamine and 4-tert-octylamino-2,6-dichloro-s-triazine, tris-(2,2,6,6-tetramethylpiperidyl)-nitrilotriacetate, tetrakis-(2,2,6,6-tetramethyl-4-piperidyl)-1,2,3,4-butanetetracarbonic acid, 1,1'(1,2-ethanediyl)-bis-(3,3,5,5-tetramethylpiperazinone).

2.7. Oxalic acid diamides, for example, 4,4'-di-octyloxy oxanilide, 2,2,-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis (3-dimethylaminopropyl)-oxalamide, 2-ethoxy-5-tert-butyl-2'-ethyloxanilide and its mixture with 2-ethoxy-2'-ethyl-5,4'-di-tert-butyloxanilide and mixtures of ortho- and para-methoxy-as well as of o- and p-ethoxy-disubstituted oxanilides.

2.8. Hydroxyphenyl-s-triazines, for example 2,6-bis-(2,4-dimethylphenyl)-4-(2-hydroxy-4-octyloxyphenyl)-s-triazine; 2,6-bis-(2,4-dimethylphenyl)-4-(2,4-dihydroxyphenyl)-s-triazine; 2,4-bis(2,4-dihydroxphenyl)-6-(4-chlorophenyl)-s-triazine; 2,4-bis [2-hydroxy-4-(2-hydroxyethoxy)phenyl]-6-(4-chlorophenyl)-s-triazine; 2-4-bis[2-hydroxy-4-(2-hydroxyethoxy)phenyl]-6-phenyl-s-triazine; 2,4-bis[2-hydroxy-4-(2-hydroxyethoxy)phenyl]-6-(2,4-dimethylphenyl)-s-triazine; 2,4-bis[2-hydroxy-4-(2-hydroxyethoxy)phenyl]-6-(4-bromophenyl)-s-triazine; 2,4-bis[2-hydroxy-4-(2-acetoxyethoxy)phenyl]-6-(4-chlorophenyl)-s-triazine, 2,4-bis(2,4-dihydroxyphenyl)-6-(2,4-dimethylphenyl)-s-triazine.

3. Metal deactivators, for example, N,N'-diphenyloxalic acid diamide, N-salicylal-N'-salicyloylhydrazine, N,N'-bis-salicyloylhydrazine, N,N'-bis-(3,5-di-tert-butyl-4-hydroxyphenylpropionyl)-hydrazine, 3-salicyloylamino-1,2,4-triazole, bis-benzylidene-oxalic acid dihydrazide.

4. Phosphites and phosphonites, for example, triphenyl phosphite, diphenylalkyl phosphites, phenyldialkyl phosphites, tri-(nonylphenyl) phosphite, trilauryl phosphite, trioctadecyl phosphite, di-stearyl-pentaerythritol diphosphite, tris-(2,4-di-tert-butylphenyl) phosphite, di-isodecylpentaerythritol diphosphite, di-(2,4-di-tert-butylphenyl)pentaerythritol diphosphite, tristearylsorbitol triphosphite, tetrakis-(2,4-di-tert-butylphenyl) 4,4'-diphenylylenediphosphonite.

5. Compounds which destroy peroxide, for example, esters of β-thiodipropionic acid, for example the lauryl, stearyl, myristyl or tridecyl esters, mercapto-benzimidazole or the zinc salt of 2-mercaptobenzimidazole, zinc dibutyl-dithiocarbamate, dioctadecyl disulfide, pentaerythritol tetrakis-(β-dodecylmercapto)-propionate.

6. Hydroxylamines, for example, N,N-dibenzylhydroxylamine, N,N-diethylhydroxylamine, N,N-dioctylhydroxylamine, N,N-dilaurylhydroxylamine, N,N-ditertradecylhydroxylamine, N,N-dihexadecylhydroxylamine, N,N-dioctadecylhydroxylamine, N-hexadecyl-N-octadecylhydroxylamine, N-heptadecyl-N-octadecylhydroxylamine, N,N-dialkylhydroxylamine derived from hydrogenated tallow amine.

7. Polyamide stabilizers, for example copper salts in combination with iodides and/or phosphorus compounds and salts of divalent manganese.

8. Basic co-stabilizers, for example, melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids for example Ca stearate, Zn stearate, Mg stearate, Na ricinoleate and K palmitate, antimony pyrocatecholate or zinc pyrocatecholate.

9. Nucleating agents, for example, 4-tert-butyl-benzoic acid, adipic acid, diphenylacetic acid.

10. Fillers and reinforcing agents, for example, calcium carbonate, silicates, glass fibers, asbestos, talc, kaolin, mica, barium sulfate, metal oxides and hydroxides, carbon black, graphite.

11.Other additives, for example, plasticizers, lubricants, emulsifiers, pigments, optical brighteners, flameproofing agents, anti-static agents, blowing agents and thio-synergists such as dilauryl thiodipropionate or distearyl thiodipropionate.

Of particular interest is the utilization of the instant derivatives in a variety of coating systems including ambient cured and acid catalyzed coating systems. In particular, the physical integrity of the coatings is maintained to a higher degree with significant reduction in loss of gloss and yellowing. Key improvements include the substantial absence of the cure retardation encountered with N-alkyl hindered amine light stabilizers; the substantial absence of flocculation and dispersion destabilization seen when N-alkyl hindered amines are utilized in certain pigmented coating systems and the absence of adhesion loss between the coating and polycarbonate substrate. Accordingly, the present invention also relates to the use of the instant compounds, optionally together with further stabilizers, for stabilizing ambient cured coatings based on alkyd resins; thermoplastic acrylic resins; acrylic alkyds; acrylic alkyd or polyester resins optionally modified with silicon, isocyanates, isocyanurates, ketimines or oxazolidines; and epoxide resins crosslinked with carboxylic acids, anhydrides, polyamines or mercaptans; and acrylic and polyester resin systems modified with reactive groups in the backbone thereof and crosslinked with epoxides; against the degradative effects of light, moisture and oxygen.

Furthermore, in their industrial uses, enamels with high solids content based on crosslinkable acrylic, polyester, urethane or alkyd resins are cured with an additional acid catalyst. Light stabilizers containing a basic nitrogen group are generally less than satisfactory in this application. Formation of a salt between the acid catalyst and the light stabilizer leads to incompatibility or insolubility and precipitation of the salt and to a reduced level of cure and to reduced light protective action and poor resistance to moisture.

These acid catalyzed stoving lacquers are based on hot crosslinkable acrylic, polyester, polyurethane, polyamide or alkyd resins. The acrylic resin lacquers, which can be stabilized against light, moisture and oxygen in accordance with the invention, are the conventional acrylic resin stoving lacquers or thermosetting resins including acrylic/melamine systems which are described, for example, in H. Kittel's "Lehrbuch der Lacke und Beschichtungen", Vol. 1 Par 2, on pages 735 and 742 (Berlin 1972), "Lackkunstharze" (1977), by H. Wagner and H.F. Sarx, on pages 229-238, and in S. Paul's "Surface Coatings: Science and Technology" (1985).

The polyester lacquers, which can be stabilized against the action of light and moisture, are the conventional stoving lacquers described e.g. in H. Wagner and H.F. Sarx, op. cit., on pages 86-99.

The alkyd resin lacquers which can be stabilized against the action of light and moisture in accordance with the invention, are the conventional stoving lacquers which are used in particular for coating automobiles (automobile finishing lacquers), for example lacquers based on alkyd/melamine resins and alkyd/acrylic/melamine resins (see H. Wagner and H. F. Sarx, op. cit., pages 99-123). Other crosslinking agents include glycoluril resins, blocked isocyanates or epoxy resins.

The acid catalyzed stoving lacquers stabilized in accordance with the invention are suitable both for metal finish coatings and solid shade finishes, especially in the case of retouching finishes, as well as various coil coating applications. The lacquers stabilized in accordance with the invention are preferably applied in the conventional manner by two methods, either by the single-coat method or by the two-coat method. In the latter method, the pigment-containing base coat is applied first and then a covering coat of clear lacquer over it.

It is also to be noted that the instant substituted hindered amines are applicable for use in non-acid catalyzed thermoset resins such as epoxy, epoxy-polyester, vinyl, alkyd, acrylic and polyester resins, optionally modified with silicon, isocyanates or isocyanurates. The epoxy and epoxy-polyester resins are crosslinked with conventional crosslinkers such as acids, acid anhydrides, amines, and the like.

Correspondingly, the epoxide may be utilized as the crosslinking agent for various acrylic or polyester resin systems that have been modified by the presence of reactive groups on the backbone structure.

To attain maximum light stability in such coatings, the concurrent use of other conventional light stabilizers can be advantageous. Examples are the aforementioned UV absorbers of the benzophenone, benzotriazole, acrylic acid derivative, or oxanilide type, or aryl-s-triazines or metal-containing light stabilizers, for example organic nickel compounds. In two-coat systems, these additional light stabilizers can be added to the clear coat and/or the pigmented base coat.

If such combinations are employed, the sum of all light stabilizers is 0.2 to 20% by weight, preferably 0.5 to 5% by weight, based on the film-forming resin.

Examples of different classes of UV absrobers which may be used in the instant compositions in conjunction with aforementioned piperidine compounds are referenced in a paper by H.J. Keller in European Polymer Journal Supplement, 1969, pp 105-132. These classes include the phenyl salicylates, the o-hydroxybenzophenones, the hydroxyxanthones, the benzoxazoles, the benzimidazoles, the oxadiazoles, the triazoles, the pyrimidines, the chinazolines, the s-trizines, the hydroxyphenyl-benzotriazoles, the alpha-cyanoacrylates and the benzoates.

Of particular value in the instant compositions are the benzotriazoles of high molecular weight and low volatility such as 2-[2-hydroxy-3,5-di(alpha,alpha-dimethylbenzyl)-phenyl] -2H-benzotriazole, 2-(2-hydroxy-3,5-di-tert-octylphenyl)-2H-benzotriazole, 2-(2-hydroxy-3-alpha,alpha-dimethylbenzyl-5-tert-octyl-phenyl)-2H-benzotriazole, 2-(2-hydroxy-3-tert-octyl-5-alpha,alpha-dimethylbenzylphenyl)-2H-benzotriazole, 2-(2-hydroxy-3,5-di-tert-amylphenyl)-2H-benzotriazole, 2-[2-hydroxy-3-tert-butyl-5-(2-(omega-hydroxy-octa-(ethyleneoxy)carbonyl)-ethylphenyl]-2H-benzotriazole, dodecylated 2-(2-hydroxy-5-methylphenyl)-2H-benzotriazole, 2-[2-hydroxy-3-tert-butyl-5-(2-octyloxycarbonyl)ethylphenyl]-2H-benzotriazole and the 5-chloro compounds corresonding to each of the above named benzotriazoles.

Most preferably the benzotriazoles useful in the instant compositions are 2-[2-hydroxy-3,5-di(alpha,alpha-dimethyl-benzyl)phenyl]-2H-benzotriazole, dodecylated 2-(2-hydroxy-5-methylphenyl)-2H-benzotriazole, 2-[2-hydroxy-3-tert-butyl-5-(2-(omega-hydroxy-octa-(ethyleneoxy) carbonyl)-ethylphenyl]-2H-benzotriazole, 2-[2-hydroxy-3-tert-butyl-5-(2-octyloxycarbonyl)ethylphenyl]-2H-benzotriazole and 5-chloro-2-[2-hydroxy-3-tert-butyl-5-(2-octyloxycarbonyl)ethylphenyl]-2H-benzotriazole.

It is also contemplated that the instant compounds will be particularly effective as stabilizers for polyolefin fibers, especially polypropylene fibers, when used in conjunction with other stabilizers selected from the group consisting of the phenolic antioxidants, hindered amine light stabilizers, organic phosphorus compounds, ultraviolet absorbers and mixtures thereof.

A preferred embodiment of the instant invention pertains to stabilized compositions comprising

(a) an acid catalyzed thermoset coating or enamel based on hot crosslinkable acrylic, polyester or alkyd resins,

(b) a NT-substituted 2,2,6,6-tetraalkylpiperidine compound of formula I and

(c) a UV absorber selected from the group consisting of the benzophenones, benzotriazoles, acrylic acid derivatives, organic nickel compounds, aryl-s-triazines and oxanilides.

Further ingredients which the enamels or coatings can contain are antioxidants, for example those of the sterically hindered phenol derivatives, phosphorus compounds, such as phosphites, phosphines or phosphonites, plasticizers, levelling assistants, hardening catalysts, thickeners, dispersants or adhesion promoters.

A further preferred embodiment of the instant invention is a stabilized composition containing components (a), (b) and (c) described above which additionally contains as component (d) a phosphite or phosphonite.

The amount of phosphite or phosphonite (d) which is used in the instant compositions is from 0.05 to 2% by weight, preferably from 0.1 to 1% by weight, based on the film forming resin. In two-coat systems, these stabilizers may be added to the clear coat and/or base coat.

Typical phosphite and phosphonites include triphenyl phosphite, diphenylalkyl phosphites, phenyldialkyl

phosphites, tri-(nonylphenyl)phosphite, trilauryl phosphite trioctadecyl phosphite, di-stearyl-pentaerythritol diphosphite, tris-(2,4-di-tert.butylphenyl) phosphite, diisodecylpentaerythritol diphosphite, di-(2,4-di-tert.butylphenyl)pentaerythritol diphosphite, tristearyl-sorbitol triphosphite, tetrakis-(2,4-di-tert.butylphenyl)-4,4'-diphenylylenediphosphonite.

The stabilizers are needed to impart greater retention of durability to the cured enamels, (as measured by 20° gloss, distinction of image, cracking or chalking); the stabilizers must not retard cure (normal bake for auto finishes at 121°C and low bake repair at 82°C (as measured by hardness, adhesion, solvent resistance and humidity resistance), the enamel should not yellow on curing and further color change on exposure to light should be minimized; the stabilizers should be soluble in the organic solvents normally used in coating applications such as methyl amyl ketone, xylene, n-hexyl acetate, alcohol and the like.

The instant hindered amine light stabilizers of formula I fulfill each of these requirements and provide alone or in combination with a UV-absorber outstanding light stabilization protection to the cured acid catalyzed thermoset enamels.

Still another preferred combination of the instant stabilizers is with a hydroxylamine in order to protect polypropylene fibers from gas fading.

The following examples are presented for the purpose of illustration only and are not to be construed to limit the nature or scope of the instant invention in any manner whatsoever.

Example 1

1-Acetyl-2,2,6,6-tetramethyl-4-chlorocarbonyloxypiperidine Hydrochloride

A solution of 19.9 grams (0.1 mol) of 1-acetyl-2,2,6,6-tetramethyl-4-hydroxypiperidine in 150 ml of dry methyl ethyl ketone is added dropwise at 0°C over a 30-minute period to a solution of 28 ml (approximately 0.4 mol) of phosgene in 150 ml of methyl ethyl ketone. The reaction mixture is stirred at 0°C for one hour, then at 20°C for eighteen hours. Excess dissolved phosgene is flushed out into an absorption trap with a stream of nitrogen. The solid precipitate is removed by filtration, washed twice with methyl ethyl ketone and dried with suction to yield 13.7 grams (52% yield) of the title compound which melts at 113-114°C.

Example 2

OO-tert-Amyl O-(1-Acetyl-2,2,6,6-tetramethylpiperidin-4-yl) Monoperoxycarbonate

A solution of 4.2 grams of sodium hydroxide in 20 ml of water is cooled to 0°C, then 7.05 grams of tert-amyl hydroperoxide (85%) in 12.5 ml of toluene is added over 20 minutes followed by the portionwise addition of 13 grams (0.05 mol) of 1-acetyl-2,2,6,6-tetramethyl-4-chlorocarbonyl oxypiperidine hydrochloride over a 30 minute period. The mixture is stirred at 0°C for two hours, then at 20°C for 18 hours. After cooling the reaction mixture to 0°C, the toluene layer is separated and washed with 25 ml of cold 2N sodium hydroxide solution, then with five 25 ml portions of water. The toluene solution is dried over anhydrous magnesium sulfate and stripped of solvent. The residue crystallized on standing and is then triturated with 10 ml of hexane, cooled and filtered to yield 10.7 grams (69% yield) of the title compound which melts at 70-73°C.

Analysis:

| | |
|---|---|
| Calcd for $C_{17}H_{31}NO_5$: | C, 62.0; H, 9.5; N, 4.3. |
| Found: | C, 62.4; H, 9.8; N, 4.2. |

Example 3

1-cyclohexyloxy-2,2,6,6-tetramethyl-4-chlorocarbonloxypiperidine Hydrochloride

Following the procedure of Example 1, 1-cyclohexyloxy-2,2,6,6-tetramethyl-4-hydroxypiperidine is reacted with phosgene to form the title compound in a yield of 80%. The product melts at 174-175°C.

Analysis:

| | |
|---|---|
| Calcd for $C_{16}H_{29}C_{12}NO_3$: | C, 54.2; H, 8.2; N, 3.9. |
| Found: | C, 54.4; H, 8.1; N, 3.8. |

Example 4

OO-tert-Amyl O-(1-Cyclohexyloxy-2,2,6,6-tetramethylpiperidin-4-yl) Monoperoxycarbonate

Following the procedure of Example 2, the intermediate prepared in Example 3 is transformed into the title compound. The product is purified by liquid chromatography to yield a viscous liquid in 52% yield.

Examples 5-8

A monomer composition comprising 25% butyl acrylate, 30% 2-hydroxyethyl acrylate, 27% butyl methacrylate, 15% styrene, and 3% acrylic acid is polymerized using different amounts of the peroxy initiator OO-tert-amyl O-(2-ethylhexyl) monoperoxycarbonate (TEAC Lupersol, Pennwalt). The various runs are carried out in refluxing xylene at 60% solids. Initiator concentrations are varied as a means of controlling polymer molecular weight.

To 100 grams of the monomer mixture is added 5.2 grams of the initiator corresponding to 0.32% "active oxygen" per 100 grams of monomers. The mixture of monomers and initiator is then pumped with a metering pump at a uniform rate over 1 62 minutes into a stirred reactior which contains 66.7 grams of xylene maintained at 135°C. The resulting solution is stirred for an additional two hours at 135°C to complete the polymerization.

The method described above is repeated using respectively 8.1; 7.3 and 6.5 parts of initiator per 100 parts of monomers.

The polymers prepared in Examples 5-8 are examined for molecular weight and Gardner viscosity values.

Examples 9-11

Using the procedure and the identical monomer mixture described in Examples 5-8 resins are prepared from peroxy initiators containing hindered amine moieties. For the 100 grams of monomer mixture, 6.5 grams of these three peroxy initiators are used respectively in Examples 9-11:

| | |
|---|---|
| Example 9 | OO-tert-amyl O-(1,2,2,6,6-pentamethylpiperidin-4-yl) monoperoxycarbonate |
| Example 10 | OO-tert-amyl O-(1-acetyl-2,2,6,6-tetramethyl-piperidin-4-yl) monoperoxycarbonate |
| Example 11 | OO-tert-amyl O-(1-cyclohexyloxy-2,2,6,6-tetramethylpiperidin-4-yl) monoperoxycarbonate |

Evaluation of the high-solids acrylic resin solutions of Examples 9-11 are essentially equivalent in molecular weight and Gardner viscosity values of the resin solutions made in Examples 5-8, particularly Example 8 using the same amount of initiator.

Example 12

The stabilized acrylic resin prepared in Example 11 is blended with sufficient unstabilized acrylic polyol, made by the same procedure of Example 5, so that in the final acrylic - melamine formulation described below there is 1% of the hindered amine acrylate present based on total resin solids.

The acrylic-melamine formulation comprises (all values are in parts by weight) 70 parts of acrylic resin as described above, 18 parts of melamine (Cymel 303, American Cyanamid), 0.51 part of sulfonic acid catalyst (Cycat 600, 70% DDBSA, American Cyanamid), 0.6 part of flow aid (FC 431 50% solids fluorocarbon, 3M) and 8.8 parts of methyl amyl ketone.

Thermoset acrylic enamels are prepared using the formulations cited above.

Pieces of steel sheeting 9.16 cm x 30.48 cm, coated with a polyester/epoxy primer, are then coated with a silver metallic base coat and finally with a clear finishing enamel. The basecoat is sprayed onto the coated sheet to a thickness of about 0.023 mm and air dried for three minutes.

The clear finishing enamel is then sprayed onto the sheet to a thickness of about 0.05 mm. After air drying for ten minutes, the coated sheets are baked for thirty minutes at 121°C. The Knoop hardness values of the baked coating is then determined.

Knoop Hardness of High Solid Acid Cured Coatings

| Light Stabilizer Present (1% by weight) | Knoop Hardness |
|---|---|
| Control (Example 8) | 9.3 |
| Copolymer of Example 11 | 9.1 |
| Copolymer of Example 11 plus 3% UV absorber* | 9.1 |
| Copolymer of Example 9 | 3.1 |

* 2-(2-hydroxy-3,5-di-tert-amylphenyl)-2H-benzotriazole

The effectiveness of cure is assessed from the Knoop hardness values. The higher numbers indicate greater hardness and better cure. The instant compounds having the N-hydrocarbyloxy group do not cause cure retardation as do compounds such as those having N-alkyl substitution.

Example 13

A thermoset acrylic enamel based on 70% by weight of an acrylic copolymer from 2-hydroxyethyl acrylate, butyl acrylate, butyl methacrylate, styrene, and acrylic acid and 30% by weight of a melamine resin in the presence of 0.6% dinonylnaphthalene disulfonic acid (based on total resin solids) is formulated to include either 1% by weight of hindered amine light stabilizers or 1% of a hindered amine light stabilizer and 3% of a benzotriazole UV absorber.

Commercially available epoxy primed 10.16 cm x 30.48 cm panels (Uniprime from Advanced Coatings Technology) are spray coated with a silver metallic basecoat to a thickness of about 0.023 mm and air dried for 3 minutes. The stabilized thermoset acrylic resin enamel is then sprayed onto the basecoat to thickness of 0.049 mm. After 15 minutes air drying, the coated panels are baked for 30 minutes at 121°C.

After storage for 1 week in an air-conditioned room, the coated panels are weathered in a QUV exposure apparatus according to ASTM G-53/77 using FS-40 bulbs.

20 Degree Gloss

| Acrylic Polymer | Hours QUV Exposure (FS-40) 0 | 925 | 1228 | 1500 | 1808 | 2419 | 3168 | 3476 | 4088 |
|---|---|---|---|---|---|---|---|---|---|
| Control (Example 8) | 93 | 86 | 88 | 82 | 57* | | | | |
| 1% Copolymer of Example 9 | 94 | 89 | 88 | 86 | 82 | 75 | 48* | | |
| 1% Copolymer of Example 11 | 93 | 89 | 91 | 86 | 85 | 67 | 54* | | |
| 1% Copolymer of Example 11 plus 3% Absorber** | 94 | 92 | 93 | 92 | 94 | 90 | 88 | 86 | 89 |

* indicates cracking
** 2-(2-hydroxy-3,5-di-tert-amylphenyl)-2H-benzotriazole

## Example 14

A two component acrylic urethane refinish coating based on a copolymer from 2-hydroxyethyl acrylate, butyl acrylate, butyl methacrylate, styrene, and acrylic acid and an aliphatic isocyanate crosslinking resin (Desmodur N-3390 from Mobay Corp) in a 1.05/1.00 ratio is formulated to include 1% by weight of hindered amine light stabilizer.

Commercially available 10.16 cm x 30.48 cm steel panels (Advanced Coatings Technology) are first primed with a commercial epoxy primer and then spray coated with a thermoplastic silver metallic basecoat to a thickness of about 0.023 mm and air dried for 5 minutes. The stabilized acrylic urethane clearcoat is then sprayed onto the basecoat to thickness of 0.049 mm. After storage for 1 month in an air-conditioned room, the coated panels are weathered in a QUV exposure apparatus according to ASTM G-53/77 using FS-40 bulbs.

20 Degree Gloss
QUV Exposure(FS-40)

| Acrylic Polymer | 0 Hours 20° Gloss | DOI* | 930 Hours 20 Gloss | DOI* |
|---|---|---|---|---|
| Control (Example 8) | 89 | 83 | 46 | 8 |
| Copolymer of Example 11 | 88 | 78 | 75 | 60 |

*DOI is Distinctness of Image.

## Example 15

### Light stabilization of Polypropylene

This example illustrates the light stabilizing effectiveness of instant stabilizers.

Polypropylene powder (Himont Profax 6501) stabilized with 0.2% by weight of n-octadecyl 3,5-di-tert-butyl-4-hydroxyhydrocinnamate is thoroughly blended with the indicated amount of additive. The blended materials are then milled on a two-roll mill at 182°C for five minutes, after which time the stabilized polypropylene is sheeted from the mill and allowed to cool. The milled polypropylene is then cut into pieces and compression molded on a hydraulic press at 250°C and 1.2 x $10^6$ Pa into 0.127 mm films. The sample is exposed in a fluorescent sunlight/black light chamber until failure. Failure is taken as the hours required to reach 0.5 carbonyl absorbance by infrared spectroscopy on the exposed films.

The time to failure for a polypropylene composition containing an instant compound as stabilizer is far longer than the time to failure for polypropylene having no such stabilizer present.

## Claims

1. A compound which is a free radical initiator which also contains a hindered amine light stabilizing moiety having low basicity, which compound has the formula I

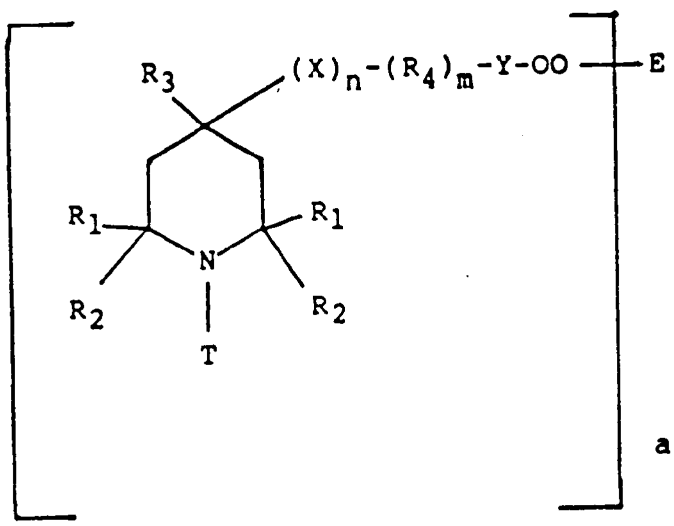

(I)

a is 1 or 2,
n and m are independently 0 or 1,
$R_1$ and $R_2$ are independently alkyl of 1 to 4 carbon atoms, or $R_1$ and $R_2$ together are pentamethylene,
$R_3$ is hydrogen, alkyl of 1 to 8 carbon atoms, alkenyl of 2 to 8 carbon atoms, alkynyl of 2 to 8 carbon atoms, cycloalkyl of 5 to 6 carbon atoms, alkoxy of 1 to 8 carbon atoms, alkoxycarbonyl of 2 to 7 carbon atoms, aryl of 6 to 10 carbon atoms, aralkyl of 7 to 15 carbon atoms, alkanoyl of 1 to 8 carbon atoms, aroyl of 7 to 16 carbon atoms, alkanoyloxy of 1 to 7 carbon atoms, or aroyloxy of 6 to 10 carbon atoms, or $R_3$ together with $R_4$ form a cyclic structure of 5 to 7 atoms,
X is -O-, -S-, -NG-, -CO-, -SO-, $-SO_2-$, -OCO-, -OSO-, $-OSO_2-$, -NG-CO-, -NHCONH- or -OCO-O- where G is hydrogen, alkyl of 1 to 8 carbon atoms, alkenyl of 2 to 8 carbon atoms, alkynyl of 2 to 8 carbon atoms, cycloalkyl of 5 to 6 carbon atoms, aryl of 6 to 10 carbon atoms, alkanoyl of 1 to 8 carbon atoms or G and $R_4$ together form a cyclic structure of 5 to 7 carbon atoms,
$R_4$ is a diradical which is alkylene of 1 to 20 carbon atoms, arylene of 6 to 10 carbon atoms, cycloalkylene of 3 to 10 carbon atoims, aralkylene of 7 to 20 carbon atoms, alkynylene of 2 to 10 carbon atoms, alkadiynylene of 4 to 10 carbon atoms, alkenylene of 3 to 11 carbon atoms, alkadienylene of 5 to 11 carbon atoms, or said diradical interrupted by one or more oxygen, sulfur or nitrogen atoms,
Y is -CO-, $-SO_2-$, $-CR_5R_6-$, -O-R-, -NG-R- or -OCO-, where $R_6$ and $R_6$ are independently alkyl of 1 to 10 carbon atoms, aryl of 6 to 10 carbon atoms, alkynyl of 2 to 10 carbon atoms, alkenyl of 2 to 8 carbon atoms or cycloalkyl of 5 to 6 carbon atoms, or $R_5$ and $R_6$ together are alkylene of 4 to 9 carbon atoms, and when E is tert-alkyl, tert-cycloalkyl or tert-aralkyl, $R_6$ is also -O-O-E, and A is di-tert-alkylene of 7 to 15 carbon atoms, di-tert-aralkylene of 12 to 20 carbon atoms, alkanedioyl of 3 to 12 carbon atoms or arene dicarbonyl of 8 to 16 carbon atoms,
E, when a is 1, is hydrogen, alkanoyl of 2 to 20 carbon atoms, aroyl of 7 to 20 carbon atoms, tert-alkyl of 4 to 12 carbon atoms, tert-cycloalkyl of 4 to 12 carbon atoms, tert-aralkyl of 9 to 15 carbon atoms, alkoxycarbonyl of 2 to 20 carbon atoms, carbamoyl, phenylcarbamoyl, alkylcarbamoyl of 2 to 13 carbon atoms, cycloalkylcarbamoyl of 4 to 13 carbon atoms, alpha-hydroxyalkyl of 2 to 10 carbon atoms, alpha-hydroxycycloalkyl of 3 to 10 carbon atoms, alkylsulfonyl of 4 to 20 carbon atoms, cycloalkylsulfonyl of 3 to 12 carbon atoms, tert-alkoxyalkyl of 4 to 20 carbon atoms, tert-alkoxycycloalkyl of 4 to 20 carbon atoms, monovalent organometal, or the radical of formula II

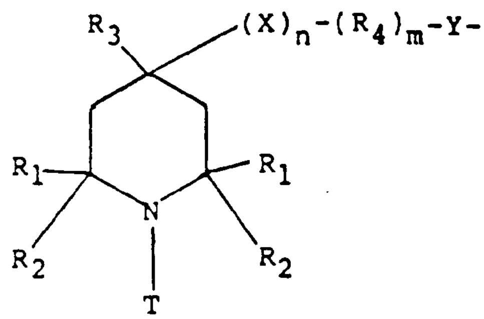

(II)

, or

E, when a is 2, is di-tert-alkylene of 7 to 15 carbon atoms, di-tert-alkenylene of 8 to 16 carbon atoms, di-tert-alkynylene of 8 to 16 carbon atoms, di-tert-aralkylene of 12 to 20 carbon atoms, alkanedioyl of 3 to 12 carbon atoms, arenedicarbonyl of 8 to 16 carbon atoms or aralkanedicarbonyl of 9 to 18 carbon atoms, and

T is formyl, -O-$T_1$ or -OCO-$T_2$, where

$T_1$ is alkyl of 1 to 36 carbon atoms, alkenyl of 2 to 18 carbon atoms, alkynyl of 2 to 18 carbon atoms, aralkyl of 7 to 15 carbon atoms, cycloalkyl of 5 to 12 carbon atoms, cycloalkenyl of 5 to 12 carbon atoms, a radical of a saturated or unsaturated bicyclic or tricyclic hydrocarbon of 7 to 12 carbon atoms, or aryl of 6 to 10 carbon atoms or said aryl substituted by alkyl, and

$T_2$ is alkyl of 1 to 18 carbon atoms, alkoxy of 1 to 18 carbon atoms, phenyl or said phenyl substituted by hydroxy, alkyl or alkoxy; or amino or said amino mono- or disubstituted by alkyl or phenyl.

2. A compound according to claim 1 wherein $R_1$ and $R_2$ are each methyl.

3. A compound according to claim 2 wherein $R_3$ is hydrogen or alkoxycarbonyl of 2 to 5 carbon atoms,

X is -O-, -S-, -NG-, -OCO-O-, -NGCOO-, -OCO- or -CO-,

$R_4$ is alkylene of 1 to 8 carbon atoms, arylene of 6 to 10 carbon atoms, aralkylene of 8 to 16 carbon atoms or cycloalkylene of 4 to 8 carbon atoms,

when a is 1, E is alkanoyl of 2 to 10 carbon atoms, aroyl of 7 to 10 carbon atoms, tert-alkyl of 4 to 8 carbon atoms, tert-aralkyl of 9 to 16 carbon atoms or the radical of formula II,

when a is 2, E is di-tert-alkylene of 8 to 12 carbon atoms, di-tert-aralkylene of 12 to 15 carbon atoms or alkanedioyl of 3 to 6 carbon atoms,

G is hydrogen, alkyl of 1 to 4 carbon atoms, cyclohexyl or phenyl,

$R_5$ is alkyl of 1 to 6 carbon atoms, aryl of 6 to 10 carbon atoms or cycloalkyl of 5 to 6 carbon atoms,

T is formyl, -$OT_1$ or -$OCOT_2$ where

$T_1$ is alkyl of 1 to 18 carbon atoms, alkenyl of 2 to 3 carbon atoms, propargyl, alpha-methylbenzyl or cyclohexyl, and

$T_2$ is alkyl of 1 to 18 carbon atoms.

4. A compound according to claim 3 wherein $R_3$ is hydrogen,

X is -O-, -NG-, -OCO-O, -NG-COO- or -OCO-,

$R_4$ is alkylene of 1 to 6 carbon atoms, phenylene, aralkylene of 9 to 12 carbon atoms or cycloalkylene of 5 to 7 carbon atoms,

Y is -CO-, -$CR_5R_5$- or -OCO-,

when a is 1, E is alkanoyl of 2 to 10 carbon atoms, benzoyl, tert-alkyl of 4 to 6 carbon atoms, tert-aralkyl of 9 to 12 carbon atoms or a radical of formula II,

when a is 2, E is di-tert-alkylene of 8 to 10 cargon atoms, di-tert-aralkylene of 12 carbon atoms or alkanedioyl of 4 to 6 carbon atoms,

G is hydrogen or alkyl of 1 to 4 carbon atoms,

$R_5$ is alkyl of 1 to 4 carbon atoms, phenyl or cyclohexyl, and

T is -$OT_1$ or -OCO-$T_2$ where $T_1$ is methyl, heptyl, octyl, nonyl or cyclohexyl, and $T_2$ is alkyl of 1 to 12 carbon atoms.

5. The compound according to claim 1 which is OO-tert-amyl O-(1-cyclohexyloxy-2,2,6,6-tetramethylpiperidin-4-yl) monoperoxycarbonate.

6. The compound according to claim 1 which is OO-tert-butyl O-(1-octyloxy-2,2,6,6-tetramethylpiperidin-4-yl) monoperoxycarbonate.

7. A process of preparing a homo- or copolymer containing a hindered amine light stabilizer moiety chemically bonded to the backbone of said polymer which process comprises polymerizing one or more ethylenically unsaturated monomer capable of being polymerized by free radicals in the presence of an effective initiating amount of a compound of formula I according to claim 1.

8. A polymer stabilized against the deleterious effects of actinic light which polymer is made according to the process according to claim 7.

9. A polymer according to claim 8 wherein the compound of formula I is OO-tert-amyl O-(1-cyclohexyloxy-2,2,6,6-tetramethylpiperidin-4-yl) monoperoxycarbonate.

10. A polymer according to claim 8 which is an acrylic resin.

11. A polymer composition stabilized against the deleterious effects of actinic light which contains an effective stabilizing amount of a stabilized polymer according to claim 8.

12. A composition according to claim 11 wherein the polymer is a coating system based on alkyd, acrylic, acrylic alkyd, polyester, epoxide, urethane, polyamide, vinyl or epoxy-polyester resins.

13. A composition according to claim 12 which contains a UV absorber or additional light stabilizer.

14. A method for stabilizing an organic material against oxidative, thermal or actinic degradation which comprises incorporating into said organic material an effective stabilizing amount of a polymer according to claim 8.

15. A composition stabilized against the deleterious effects of actinic light which comprises
(a) a polymer, and
(b) an effective stabilizing amount of a compound according to claim 1.

16. A composition according to claim 15 wherein the polymer is a polyolefin.

17. A composition according to claim 16 wherein the polyolefin is polypropylene.

18. A composition according to claim 15 wherein the polymer is a coating system based on alkyd, acrylic, acrylic-alkyd, polyester, epoxide, urethane, polyamide, vinyl or epoxy-polyester resins.

19. A composition according to claim 15 which contains a UV absorber or additional light stabilizer.

20. A composition according to claim 15 wherein component (b) is OO-tert-amyl O-(1-cyclohexyloxy-2,2,6,6-tetramethylpiperidin -4-yl) monoperoxycarbonate.

21. A composition according to claim 15 wherein component (b) is OO-tert-butyl O-(1-octyloxy-2,2,6,6-tetramethylpiperidin-4-yl) monoperoxycarbonate.

22. A composition according to claim 15 wherein the polymer is an unsaturated elastomer which is polybutadiene, polyisoprene, styrene-butadiene copolymer or block copolymer, ethylene-propylene terpolymer, isoprene-isobutylene copolymer, acrylonitrile-butadiene copolymer, or styrene-isoprene copolymer or block copolymer.

23. A composition according to claim 22 wherein the elastomer is styrene-butadiene copolymer or block copolymer, styrene-isoprene copolymer or block copolymer or polybutadiene.

24. A composition according to claim 15 wherein component (b) is grafted to component (a).

25. A method for stabilizing an organic material against oxidative, thermal or actinic degradation which comprises incorporating into said organic material an effective stabilizing amount of a compound according to claim 1.

## Patentansprüche

1. Eine Verbindung, welche ein freie Radikale-Initiator ist, die auch einen gehinderte Amin-lichtstabilisierenden Teil mit niedriger Basizität enthält, wobei diese Verbindung die Formel I hat,

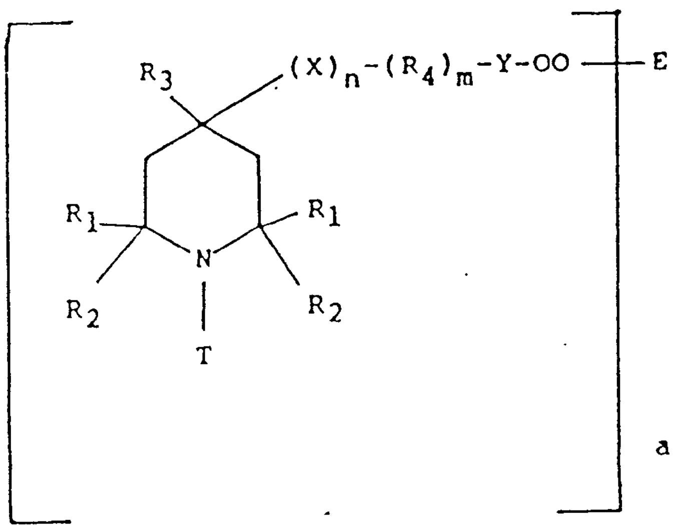

(I)

worin
a 1 oder 2 ist,
n und m unabhängig 0 oder 1 sind,
$R_1$ und $R_2$ sind unabhängig Alkyl mit 1 bis 4 Kohlenstoffatomen, oder $R_1$ und $R_2$ sind zusammen Pentamethylen,
$R_3$ ist Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Cycloalkyl mit 5 bis 6 Kohlenstoffatomen, Alkoxy mit 1 bis 8 Kohlenstoffatomen, Alkoxycarbonyl mit 2 bis 7 Kohlenstoffatomen, Aryl mit 6 bis 10 Kohlenstoffatomen, Aralkyl mit 7 bis 15 Kohlenstoffatomen, Alkanoyl mit 1 bis 8 Kohlenstoffatomen, Aroyl mit 7 bis 16 Kohlenstoffatomen, Alkanoyloxy mit 1 bis 7 Kohlenstoffatomen, oder Aroyloxy mit 6 bis 10 Kohlenstoffatomen, oder $R_3$ bildet zusammen mit $R_4$ eine cyclische Struktur mit 5 bis 7 Atomen,
X ist -O-, -S-, -NG-, -CO-, -SO-, $-SO_2-$, -OCO-, -OSO-, $-OSO_2-$, -NG-CO-, -NHCONH- oder -OCO-O-, wobei G Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Cycloalkyl mit 5 bis 6 Kohlenstoffatomen, Aryl mit 6 bis 10 Kohlenstoffatomen, Alkanoyl mit 1 bis 8 Kohlenstoffatomen ist, oder G und $R_4$ bilden zusammen eine cyclische Struktur mit 5 bis 7 Kohlenstoffatomen,
$R_4$ ist ein Diradikal bzw. zweiwertiger Rest, der Alkylen mit 1 bis 20 Kohlenstoffatomen, Arylen mit 6 bis 10 Kohlenstoffatomen, Cycloalkylen mit 3 bis 10 Kohlenstoffatomen, Aralkylen mit 7 bis 20 Kohlenstoffatomen, Alkinylen mit 2 bis 10 Kohlenstoffatomen, Alkadiinylen mit 4 bis 10 Kohlenstoffatomen, Alkenylen mit 3 bis 11 Kohlenstoffatomen, Alkadienylen mit 5 bis 11 Kohlenstoffatomen ist, oder dieser zweiwertige Rest ist unterbrochen durch ein oder mehrere Sauerstoff-, Schwefel- oder Stickstoffatome,
Y ist -CO-, $-SO_2-$, $-CR_5R_5-$, -O-R-, -NG-R oder -OCO-, worin $R_5$ und $R_6$ unabhängig voneinander Alkyl mit 1 bis 10 Kohlenstoffatomen, Aryl mit 6 bis 10 Kohlenstoffatomen, Alkinyl mit 2 bis 10 Kohlenstoffatomen, Alkenyl mit 2 bis 8 Kohlenstoffatomen oder Cycloalkyl mit 5 bis 6 Kohlenstoffatomen sind, oder $R_5$ und $R_6$ sind zusammen Alkylen mit 4 bis 9 Kohlenstoffatomen, und wenn E tert.-Alkyl, tert.-Cycloalkyl oder tert.-Aralkyl ist, ist $R_6$ auch -O-O-E, und R ist Di-tert.-alkylen mit 7 bis 15 Kohlenstoffatomen, Di-tert.-aralkylen mit 12 bis 20 Kohlenstoffatomen, Alkandioyl mit 3 bis 12 Kohlenstoffatomen oder Aren-dicarbonyl mit 8 bis 16 Kohlenstoffatomen,

E ist, wenn a=1 ist, Wasserstoff, Alkanoyl mit 2 bis 20 Kohlenstoffatomen, Aroyl mit 7 bis 20 Kohlenstoffatomen, tert.-Alkyl mit 4 bis 12 Kohlenstoffatomen, tert.-Cycloalkyl mit 4 bis 12 Kohlenstoffatomen, tert.-Aralkyl mit 9 bis 15 Kohlenstoffatomen, Alkoxycarbonyl mit 2 bis 20 Kohlenstoffatomen, Carbamoyl, Phenylcarbamoyl, Alkylcarbamoyl mit 2 bis 13 Kohlenstoffatomen, Cycloalkylcarbamoyl mit 4 bis 13 Kohlenstoffatomen, alpha-Hydroxyalkyl mit 2 bis 10 Kohlenstoffatomen, alpha-Hydroxycycloalkyl mit 3 bis 10 Kohlenstoffatomen, Alkylsulfonyl mit 4 bis 20 Kohlenstoffatomen, Cycloalkylsulfonyl mit 3 bis

12 Kohlenstoffatomen, tert.-Alkoxyalkyl mit 4 bis 20 Kohlenstoffatomen, tert.-Alkoxycycloalkyl mit 4 bis 20 Kohlenstoffatomen, einwertiges Organometall oder der Rest der Formel II

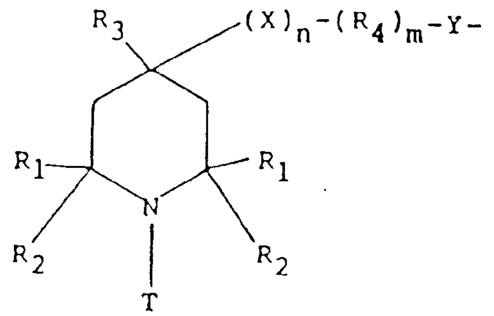

(II)

oder E ist, wenn a = 2 ist, Di-tert.-alkylen mit 7 bis 15 Kohlenstoffatomen, Di-tert.-alkenylen mit 8 bis 16 Kohlenstoffatomen, Di-tert.-alkinylen mit 8 bis 16 Kohlenstoffatomen, Di-tert.-aralkylen mit 12 bis 20 Kohlenstoffatomen, Alkandioyl mit 3 bis 12 Kohlenstoffatomen, Arendicarbonyl mit 8 bis 16 Kohlenstoffatomen oder Aralkandicarbonyl mit 9 bis 18 Kohlenstoffatomen, und

T ist Formyl, $-O-T_1$ oder $-OCO-T_2$, wobei

$T_1$ Alkyl mit 1 bis 36 Kohlenstoffatomen, Alkenyl mit 2 bis 18 Kohlenstoffatomen, Alkinyl mit 2 bis 18 Kohlenstoffatomen, Aralkyl mit 7 bis 15 Kohlenstoffatomen, Cycloalkyl mit 5 bis 12 Kohlenstoffatomen, Cycloalkenyl mit 5 bis 12 Kohlenstoffatomen, ein Rest eines gesättigten oder ungesättigten bicyclischen oder tricyclischen Kohlenwasserstoffs mit 7 bis 12 Kohlenstoffatomen oder Aryl mit 6 bis 10 Kohlenstoffatomen oder dieses Aryl substituiert durch Alkyl ist, und

$T_2$ Alkyl mit 1 bis 18 Kohlenstoffatomen, Alkoxy mit 1 bis 18 Kohlenstoffatomen, Phenyl oder dieses Phenyl substituiert durch Hydroxy, Alkyl oder Alkoxy ist; oder Amino oder dieses Amino mono- oder disubstituiert durch Alkyl oder Phenyl ist.

**2.** Eine Verbindung gemäß Anspruch 1, worin $R_1$ und $R_2$ jeweils Methyl sind.

**3.** Eine Verbindung gemäß Anspruch 2, worin $R_3$ Wasserstoff oder Alkoxycarbonyl mit 2 bis 5 Kohlenstoffatomen ist,

X ist -O-, -S-, -NG-, -OCO-O-, -NGCOO-, -OCO- oder-CO-,

$R_4$ ist Alkylen mit 1 bis 8 Kohlenstoffatomen, Arylen mit 6 bis 10 Kohlenstoffatomen, Aralkylen mit 8 bis 16 Kohlenstoffatomen oder Cycloalkylen mit 4 bis 8 Kohlenstoffatomen,

wenn a = 1 ist, ist E Alkanoyl mit 2 bis 10 Kohlenstoffatomen, Aroyl mit 7 bis 10 Kohlenstoffatomen, tert.-Alkyl mit 4 bis 8 Kohlenstoffatomen, tert.-Aralkyl mit 9 bis 16 Kohlenstoffatomen oder der Rest der Formel II,

wenn a = 2 ist, ist E Di-tert.-alkylen mit 8 bis 12 Kohlenstoffatomen, Di-tert.-aralkylen mit 12 bis 15 Kohlenstoffatomen oder Alkandioyl mit 3 bis 6 Kohlenstoffatomen,

G ist Wasserstoff, Alkyl mit 1 bis 4 Kohlenstoffatomen, Cyclohexyl oder Phenyl,

$R_5$ ist Alkyl mit 1 bis 6 Kohlenstoffatomen, Aryl mit 6 bis 10 Kohlenstoffatomen oder Cycloalkyl mit 5 bis 6 Kohlenstoffatomen,

T ist Formyl, $-OT_1$ oder $OCOT_2$, wobei

$T_1$ Alkyl mit 1 bis 18 Kohlenstoffatomen, Alkenyl mit 2 bis 3 Kohlenstoffatomen, Propargyl, alpha-Methylbenzyl oder Cyclohexyl ist, und

$T_2$ Alkyl mit 1 bis 18 Kohlenstoffatomen ist.

**4.** Eine Verbindung gemäß Anspruch 3, worin $R_3$ Wasserstoff ist

X ist -O-, -NG-, -OCO-O, -NG-COO- oder -OCO-,

$R_4$ ist Alkylen mit 1 bis 6 Kohlenstoffatomen, Phenylen, Aralkylen mit 9 bis 12 Kohlenstoffatomen oder Cycloalkylen mit 5 bis 7 Kohlenstoffatomen,

Y ist -CO-, $-CR_5R_6-$ oder -OCO-,

wenn a = 1 ist, ist E Alkanoyl mit 2 bis 10 Kohlenstoffatomen, Benzoyl, tert.-Alkyl mit 4 bis 6 Kohlenstoffatomen, tert.-Aralkyl mit 9 bis 12 Kohlenstoffatomen oder ein Rest der Formel II,

wenn a = 2 ist, ist E Di-tert.-alkylen mit 8 bis 10 Kohlenstoffatomen, Di-tert.-aralkylen mit 12 Kohlenstoffatomen oder Alkandioyl mit 4 bis 6 Kohlenstoffatomen,

G ist Wasserstoff oder Alkyl mit 1 bis 4 Kohlenstoffatomen,
$R_5$ ist Alkyl mit 1 bis 4 Kohlenstoffatomen, Phenyl oder Cyclohexyl, und
T ist $-OT_1$ oder $-OCO-T_2$, worin $T_1$ Methyl, Heptyl, Octyl, Nonyl oder Cyclohexyl ist, und $T_2$ ist Alkyl mit 1 bis 12 Kohlenstoffatomen.

**5.** Die Verbindung gemäß Anspruch 1, welche ist OO-tert.-Amyl O-(1-Cyclohexyloxy-2,2,6,6-tetramethylpiperidin-4-yl)-monoperoxycarbonat.

**6.** Die Verbindung gemäß Anspruch 1, welche ist OO-tert.-Butyl O-(1-Octyloxy-2,2,6,6-tetramethylpiperidin-4-yl)-monoperoxycarbonat.

**7.** Verfahren zur Herstellung eines Homo- oder Copolymeren enthaltend ein gehindertes Amin-Lichtstabilisator-Teil, chemisch gebunden an die Hauptkette dieses Polymeren, wobei dieses Verfahren das Polymerisieren eines oder mehrerer ethylenisch ungesättigter Monomerer umfaßt, das in der Lage ist, durch freie Radikale in Anwesenheit einer wirksamen Initiatormenge einer Verbindung der Formel I gemäß Anspruch 1 polymerisiert zu werden.

**8.** Polymeres, stabilisiert gegen die schädlichen Wirkungen von aktinischem Licht, wobei dieses Polymere nach dem Verfahren gemäß Anspruch 7 hergestellt ist.

**9.** Polymeres gemäß Anspruch 8, worin die Verbindung der Formel I ist OO-tert.-Amyl O-(1-Cyclohexyloxy-2,2,6,6-tetramethylpiperidin-4-yl)-monoperoxycarbonat.

**10.** Polymeres gemäß Anspruch 8, das ein Acrylharz ist.

**11.** Polymerzusammensetzung, stabilisiert gegen die schädlichen Wirkungen von aktinischem Licht, welche eine wirksame stabilisierende Menge eines stabilisierten Polymeren gemäß Anspruch 8 enthält.

**12.** Zusammensetzung gemäß Anspruch 11, worin das Polymere ein Beschichtungssystem auf Basis von Alkyd-, Acryl-, Acrylalkyd-, Polyester-, Epoxid-, Urethan-, Polyamid-, Vinyl- oder Epoxy-Polyester-Harzen ist.

**13.** Zusammensetzung gemäß Anspruch 12, welche einen UV-Absorber oder zusätzlichen Lichtstabilisator enthält.

**14.** Methode zum Stabilisieren eines organischen Materials gegen oxidativen, thermischen oder aktinischen Abbau, welche das Einverleiben einer wirksamen stabilisierenden Menge eines Polymeren gemäß Anspruch 8 in dieses organische Material umfaßt.

**15.** Zusammensetzung, stabilisiert gegen die schädlichen Wirkungen von aktinischem Licht, umfassend
(a) ein Polymeres, und
(b) eine wirksame stabilisierende Menge einer Verbindung gemäß Anspruch 1.

**16.** Zusammensetzung gemäß Anspruch 15, worin das Polymere ein Polyolefin ist.

**17.** Zusammensetzung gemäß Anspruch 16, worin das Polyolefin Polypropylen ist.

**18.** Zusammensetzung gemäß Anspruch 15, worin das Polymere ein Überzugs- bzw. Beschichtungssystem auf Basis von Alkyd-, Acryl-, Acryl-Alkyd-, Polyester-, Epoxid-, Urethan-, Polyamid-, Vinyl- oder Epoxy-Polyester-Harzen ist.

**19.** Zusammensetzung gemäß Anspruch 15, welche einen UV-Absorber oder zusätzlichen Lichtstabilisator enthält.

**20.** Zusammensetzung gemäß Anspruch 15, worin die Komponente (b) OO-tert.-Amyl O-(1-Cyclohexyloxy-2,2,6,6-tetramethyl-piperidin-4-yl)-monoperoxycarbonat ist.

**21.** Zusammensetzung gemäß Anspruch 15, worin die Komponente (b) OO-tert.-Butyl O-(1-Octyloxy-2,2,6,6-tetramethylpiperidin-4-yl)-monoperoxycarbonat ist.

**22.** Zusammensetzung gemäß Anspruch 15, worin das Polymere ein ungesättigtes Elastomeres ist, welches

Polybutadien, Polyisopren, Styrol/Butadien-Copolymeres oder -Blockcopolymeres, Ethylen/Propylen-Terpolymeres, Isopren/Isobutylen-Copolymeres, Acrylnitril/Butadien-Copolymeres oder Styrol/Isopren-Copolymeres oder -Blockcopolymeres ist.

23. Zusammensetzung gemäß Anspruch 22, worin das Elastomere Styrol/Butadien-Copolymeres oder -Blockcopolymeres, Styrol/Isopren-Copolymeres oder -Blockcopolymeres oder Polybutadien ist.

24. Zusammensetzung gemäß Anspruch 15, worin die Komponente (b) auf die Komponente (a) gepfropft ist.

25. Methode zum Stabilisieren eines organischen Materials gegen oxidativen, thermischen oder aktinischen Abbau, umfassend das Einverleiben einer wirksamen stabilisierenden Menge einer Verbindung gemäß Anspruch 1 in dieses organische Material.

## Revendications

1. Composé qui est un initiateur à radicaux libres, contenant également un groupement stabilisant à la lumière du type amine à empêchement stérique ayant une faible basicité, lequel composé répond à la formule I

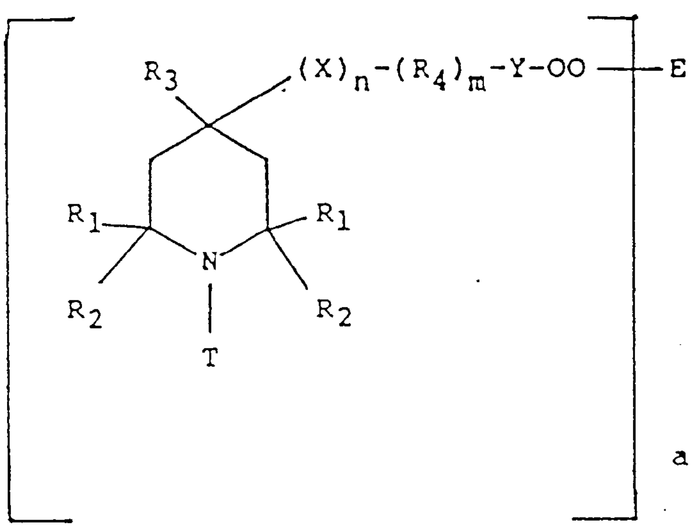

(I)

a est 1 ou 2,

n et m sont chacun indépendamment 0 ou 1,

$R_1$ et $R_2$ sont chacun indépendamment un groupe alkyle de 1 à 4 atomes de carbone, ou bien $R_1$ et $R_2$ forment ensemble un groupe pentaméthylène,

$R_3$ est l'hydrogène, un groupe alkyle de 1 à 8 atomes de carbone, alcényle de 2 à 8 atomes de carbone, alcynyle de 2 à 8 atomes de carbone, cycloalkyle de 5 ou 6 atomes de carbone, alcoxy de 1 à 8 atomes de carbone, alcoxycarbonyle de 2 à 7 atomes de carbone, aryle de 6 à 10 atomes de carbone, aralkyle de 7 à 15 atomes de carbone, alcanoyle de 1 à 8 atomes de carbone, aroyle de 7 à 16 atomes de carbone, alcanoyloxy de 1 à 7 atomes de carbone ou aroyloxy de 6 à 10 atomes de carbone, ou bien $R_3$ forme avec $R_4$ une structure cyclique de 5 à 7 atomes de carbone,

X est -O-, -S-, -NG-, -CO-, -SO-, $-SO_2-$, -OCO-, -OSO-, $-OSO_2-$, -NG-CO-, -NHCONH- ou -OCO-O- où G est l'hydrogène, un groupe alkyle de 1 à 8 atomes de carbone, alcényle de 2 à 8 atomes de carbone, alcynyle de 2 à 8 atomes de carbone, cycloalkyle de 5 ou 6 atomes de carbone, aryle de 6 à 10 atomes de carbone, alcanoyle de 1 à 8 atomes de carbone, ou bien G et $R_4$ forment ensemble une structure cyclique de 5 à 7 atomes de carbone,

$R_4$ est un radical divalent qui est un groupe alkylène de 1 à 20 atomes de carbone, arylène de 6 à 10 atomes de carbone, cycloalkylène de 3 à 10 atomes de carbone, aralkylène de 7 à 20 atomes de carbone, alcynylène de 2 à 10 atomes de carbone, alcadiynylène de 4 à 10 atomes de carbone, alcénylène de 3 à 11 atomes de carbone, alcadiénylène de 5 à 11 atomes de carbone, ou bien ledit radical divalent interrompu par un ou plusieurs atomes d'oxygène, de soufre ou d'azote,

Y est -CO-, $-SO_2-$, $-CR_5R_6-$, -O-R-, -NG-R- ou -OCO-, où $R_5$ et $R_5$ sont chacun indépendamment un groupe alkyle de 1 à 10 atomes de carbone, aryle de 6 à 10 atomes de carbone, alcynyle de 2 à 10 atomes de carbone, alcényle de 2 à 8 atomes de carbone ou cycloalkyle de 5 ou 6 atomes de carbone, ou bien $R_5$ et $R_5$ forment ensemble un groupe alkylène de 4 à 9 atomes de carbone, et si E est un groupe *tert*-alkyle, *tert*-cycloalkyle ou *tert*-aralkyle, $R_5$ peut être également -O-O-E, et R est un groupe di-*tert*-alkylène de 7 à 15 atomes de carbone, di-*tert*-aralkylène de 12 à 20 atomes de carbone, alcanedioyle de 3 à 12 atomes de carbone ou arène-dicarbonyle de 8 à 16 atomes de carbone,

si a est 1, E est l'hydrogène, un groupe alcanoyle de 2 à 20 atomes de carbone, aroyle de 7 à 20 atomes de carbone, *tert*-alkyle de 4 à 12 atomes de carbone, *tert*-cycloalkyle de 4 à 12 atomes de carbone, *tert*-aralkyle de 9 à 15 atomes de carbone, alcoxycarbonyle de 2 à 20 atomes de carbone, carbamoyle, phénylcarbamoyle, alkylcarbamoyle de 2 à 13 atomes de carbone, cycloalkylcarbamoyle de 4 à 13 atomes de carbone, alpha-hydroxyalkyle de 2 à 10 atomes de carbone, alpha-hydroxycycloalkyle de 3 à 10 atomes de carbone, alkylsulfonyle de 4 à 20 atomes de carbone, cycloalkylsulfonyle de 3 à 12 atomes de carbone, *tert*-alcoxyalkyle de 4 à 20 atomes de carbone, *tert*-alcoxycycloalkyle de 4 à 20 atomes de carbone, un radical organo-métallique monovalent ou le radical de formule II

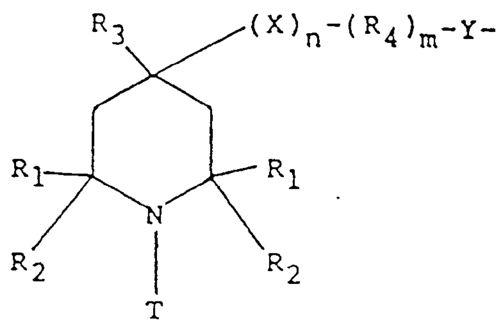

(II)

ou bien,

si a est 2, E est un groupe di-*tert*-alkylène de 7 à 15 atomes de carbone, di-*tert*-alcénylène de 8 à 16 atomes de carbone, di-*tert*-alcynylène de 8 à 16 atomes de carbone, di-*tert*-aralkylène de 12 à 20 atomes de carbone, alcane-dioyle de 3 à 12 atomes de carbone, arène-dicarbonyle de 8 à 16 atomes de carbone ou aralcane-dicarbonyle de 9 à 18 atomes de carbone, et

T est le groupe formyle, $O-T_1$ ou $-OCO-T_2$, où

$T_1$ est un groupe alkyle de 1 à 36 atomes de carbone, alcényle de 2 à 18 atomes de carbone, alcynyle de 2 à 18 atomes de carbone, aralkyle de 7 à 15 atomes de carbone, cycloalkyle de 5 à 12 atomes de carbone, cycloalcényle de 5 à 12 atomes de carbone, un radical d'un hydrocarbure bicyclique ou tricyclique saturé ou insaturé de 7 à 12 atomes de carbone, ou un groupe aryle de 6 à 10 atomes de carbone ou ledit groupe aryle substitué par un groupe alkyle, et

$T_2$ est un groupe alkyle de 1 à 18 atomes de carbone, alcoxy de 1 à 18 atomes de carbone, phényle ou phényle à substitution hydroxyle, alkyle ou alcoxy ; ou un groupe amino ou amino substitué par un ou deux groupes alkyle ou phényle.

2. Composé selon la revendication 1, dans lequel $R_1$ et $R_2$ sont chacun un groupe méthyle.

3. Composé selon la revendication 2, dans lequel $R_3$ est l'hydrogène ou un groupe alcoxycarbonyle de 2 à 5 atomes de carbone,

X est -O-, -S-, -NG-, -OCO-O-, -NGCOO-, -OCO- ou -CO-,

$R_4$ est un groupe alkylène de 1 à 8 atomes de carbone, arylène de 6 à 10 atomes de carbone, aralkylène de 8 à 16 atomes de carbone ou cycloalkylène de 4 à 8 atomes de carbone,

si a est 1, E est un groupe alcanoyle de 2 à 10 atomes de carbone, aroyle de 7 à 10 atomes de carbone, *tert*- alkyle de 4 à 8 atomes de carbone, *tert*-aralkyle de 9 à 16 atomes de carbone ou le radical de formule II,

si a est 2, E est un groupe di-*tert*-alkylène de 8 à 12 atomes de carbone, di-*tert*-aralkylène de 12 à 15 atomes de carbone ou alcanedioyle de 3 à 6 atomes de carbone,

G est l'hydrogène, un groupe alkyle de 1 à 4 atomes de carbone, cyclohexyle ou phényle,

$R_5$ est un groupe alkyle de 1 à 6 atomes de carbone, aryle de 6 à 10 atomes de carbone ou cycloalkyle de 5 ou 6 atomes de carbone,

T est un groupe formyle, $-OT_1$ ou $-OCOT_2$ où

$T_1$ est un groupe alkyle de 1 à 18 atomes de carbone, alcényle de 2 ou 3 atomes de carbone, propargyle, alpha-méthylbenzyle ou cyclohexyle, et

$T_2$ est un groupe alkyle de 1 à 18 atomes de carbone.

**4.** Composé selon la revendication 3, dans lequel $R_3$ est l'hydrogène,

X est -O-, -NG-, -OCO-O-, -NG-COO- ou -OCO-,

$R_4$ est un groupe alkylène de 1 à 6 atomes de carbone, phénylène, aralkylène de 9 à 12 atomes de carbone ou cycloalkylène de 5 à 7 atomes de carbone,

Y est -CO-, $-CR_5R_6-$ ou -OCO-,

si a est 1, E est un groupe alcanoyle de 2 à 10 atomes de carbone, benzoyle, *tert*-alkyle de 4 à 6 atomes de carbone, *tert*-aralkyle de 9 à 12 atomes de carbone ou un radical de formule II,

si a est 2, E est un groupe di-*tert*-alkylène de 8 à 10 atomes de carbone, di-*tert*-aralkylène de 12 atomes de carbone ou alcanedioyle de 4 à 6 atomes de carbone,

G est l'hydrogène ou un groupe alkyle de 1 à 4 atomes de carbone,

$R_5$ est un groupe alkyle de 1 à 4 atomes de carbone, phényle ou cyclohexyle, et

T est $-OT_1$ ou $-OCO-T_2$ où $T_1$ est un groupe méthyle, heptyle, octyle, nonyle ou cyclohexyle et $T_2$ est un groupe alkyle de 1 à 12 atomes de carbone.

**5.** Composé selon la revendication 1, qui est le monoperoxycarbonate de O-(1-cyclohexyloxy-2,2,6,6-tétraméthylpipéridine-4-yle) et de OO-*tert*-amyle.

**6.** Composé selon la revendication 1, qui est le monoperoxycarbonate de O-(1-octyloxy-2,2,6,6-tétraméthylpipéridine-4-yle) et de OO-*tert*-butyle.

**7.** Procédé de préparation d'un homo- ou copolymère contenant un groupement stabilisant à la lumière du type amine à empêchement stérique lié chimiquement au squelette dudit polymère, lequel procédé consiste à polymériser un ou plusieurs monomères éthyléniquement insaturés capables d'être polymérisés par des radicaux libres en présence d'une quantité initiatrice efficace d'un composé de formule I selon la revendication 1.

**8.** Polymère stabilisé contre les effets nuisibles de la lumière actinique, lequel polymère est préparé selon le procédé selon la revendication 7.

**9.** Polymère selon la revendication 8, dans lequel le composé de formule I est le monoperoxycarbonate de O-(1-cyclohexyloxy-2,2,6,6-tétraméthylpipéridine-4-yle) et de OO-*tert*-amyle.

**10.** Polymère selon la revendication 8, qui est une résine acrylique.

**11.** Composition de polymère stabilisée contre les effets nuisibles de la lumière actinique, qui contient une quantité à effet stabilisant d'un polymère stabilisé selon la revendication 8.

**12.** Composition selon la revendication 11, dans laquelle le polymère est un système de revêtement à base de résine alkyde, acrylique, alkyde-acrylique, polyester, époxy, uréthanne, polyamide, vinylique ou époxy-polyester.

**13.** Composition selon la revendication 12, qui contient un absorbant d'UV ou un stabilisant à la lumière supplémentaire.

**14.** Procédé pour stabiliser une matière organique contre la dégradation par l'oxydation, la chaleur ou la lumière actinique, qui consiste à incorporer à ladite matière organique une quantité à effet stabilisant d'un polymère selon la revendication 8.

**15.** Composition stabilisée contre les effets nuisibles de la lumière actinique, qui comprend

(a) un polymère, et

(b) une quantité à effet stabilisant d'un composé selon la revendication 1.

**16.** Composition selon la revendication 15, dans laquelle le polymère est une polyoléfine.

**17.** Composition selon la revendication 16, dans laquelle la polyoléfine est le polypropylène.

**18.** Composition selon la revendication 15, dans laquelle le polymère est un système de revêtement à base de résine alkyde, acrylique, alkyde-acrylique, polyester, époxy, uréthanne, polyamide, vinylique ou époxy-polyester.

**19.** Composition selon la revendication 15, qui contient un absorbant d'UV ou un stabilisant à la lumière supplémentaire.

**20.** Composition selon la revendication 15, dans laquelle le composant (b) est le monoperoxycarbonate de O-(1-cyclohexyloxy-2,2,6,6-tétraméthylpipéridine-4-yle) et de OO-*tert*-amyle.

**21.** Composition selon la revendication 15, dans laquelle le composant (b) est le monoperoxycarbonate de O-(1-octyloxy-2,2,6,6-tétraméthylpipéridine-4-yle) et de OO-*tert*-butyle.

**22.** Composition selon la revendication 15, dans laquelle le polymère est un élastomère insaturé qui est un polybutadiène, un polyisoprène, un copolymère ou copolymère séquencé styrène-butadiène, un terpolymère éthylènepropylène-diène, un copolymère isoprène-isobutylène, un copolymère acrylonitrile-butadiène ou un copolymère ou copolymère séquencé styrène-isoprène.

**23.** Composition selon la revendication 22, dans laquelle l'élastomère est un copolymère ou copolymère séquencé styrène-butadiène, un copolymère ou copolymère séquencé styrène-isoprène ou un polybutadiène.

**24.** Composition selon la revendication 15, dans laquelle le composant (b) est greffé sur le composant (a).

**25.** Procédé pour stabiliser une matière organique contre la dégradation par l'oxydation, la chaleur ou la lumière actinique, qui consiste à incorporer à ladite matière organique une quantité à effet stabilisant d'un composé selon la revendication 1.